# EUROPEAN PATENT APPLICATION

(11) **EP 0 737 689 A1**
(43) Date of publication of application: **16.10.1996**
(21) Application number: 95903001.6
(22) Date of filing: 20.12.1994
(51) Int. Cl.: C07J 73/00, A61K 31/56

(54) **NOVEL 6-SUBSTITUTED OXA- OR AZASTEROID COMPOUND**

(30) Priority: 22.12.1993 JP 345535/93
(71) Applicant: TEIKOKU HORMONE MFG. CO., LTD., Tokyo 107 (JP)
(72) Inventor: KOIZUMI, Naoyuki, Kanagawa 228 (JP); TAKEGAWA, Shigehiro 2-10-32-401, Shimokodanaka, Kanagawa 211 (JP); IWASHITA, Shigeki Teikoku Zoki Syataku, Kanagawa 211 (JP); KAWACHI, Tomoko Greenheights Yuki 10C, Tokyo 206 (JP); DOGUCHI, Koji Teikoku Zoki Syataku, Kanagawa 211 (JP); HONMA, Seijiro 2-4-19, Tachibanadai, Kawanaga 227 (JP); NAKAYAMA, Mitsuya Teikoku Zoki Ryo, Kanagawa 211 (JP); MIEDA, Mamoru, Kanagawa 243-04 (JP); TAKAHASHI, Hiroo, Kanagawa 229 (JP); SHIBATA, Kenyu, Tokyo 206 (JP)
(74) Representative: Weisert, Annekäte, Dipl.-Ing. Dr.-Ing.
(86) International application number: JP9402151
(87) International publication number: WO9517417

(57) **Abstract**

6-Substituted oxa- or azasteroid compounds represented by the formula 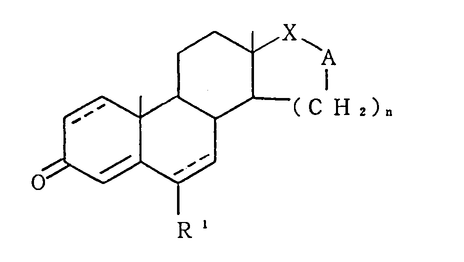 have an excellent aromatase inhibition action, and are useful for prophylaxis or treatment of diseases caused by estrogens such as, for example, breast cancer, uterine cancer and prostatic hypertrophy.

The characteristics of such a compound is that an oxygen or nitrogen atom is introduced in the D ring portion, and the 6-position of the steroid skeleton (substituent R¹) is substituted with -S-R² wherein R² represents a hydrogen atom, a lower alkyl group, a cyano group or an acyl group; -O-R³ wherein R³ represents a hydrogen atom, a lower alkyl group or an acyl group; an azido group or an amino group.

## Description

### Technical Field

This invention relates to a novel 6-substituted oxa- or azasteroid compound having an aromatase inhibition action, and, more detailedly, relates to a steroid compound represented by the formula 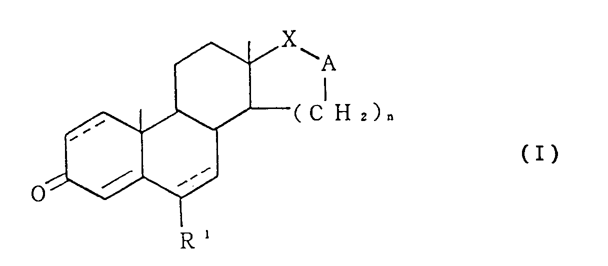 wherein
- R¹: represents -S-R², -O-R³ , an azido group or an amino group, and herein
- R²: represents a hydrogen atom, a lower alkyl group, a cyano group or an acyl group,
- R³: represents a hydrogen atom, a lower alkyl group or an acyl group,
- X: represents C = O or CH₂ ,
- A: represents O or NH,
- n: represents 1 or 2, and
the broken line between the 1- and
2-positions of the steroid skeleton means that a double bond can optionally exist there, the broken line between the 6- and 7-positions means that a double bond can optionally exist when R¹ is -O-R³ and R³ represents a lower alkyl group, and in other cases than the above, the bond between the 6- and 7-positions is meant to be a single bond,
provided that when A represents NH, X is assumed to represent C = O.

### Background Art

Biosynthesis of an estrogen is conducted by that an androgen is aromatized through its oxidation and elimination of formic acid by an enzyme called aromatase. Therefore, it has been surmised that if the action of aromatase can effectively be inhibited, it will be useful for treatment of diseases caused by estrogens, and according to the idea, it is already made clear that several aromatase inhibitors are useful for treatment of breast cancer or prostatic hypertrophy.

Further, aromatase inhibitors are also useful for treatment of other diseases caused by estrogens, such as, for example, uterine cancer, ovarian cancer, endometriosis, male gynecomastia and male infertility on aspermia.

As known steroidal aromatase inhibitors, there have, for example, been known testolactone (The MERCK INDEX, tenth edition, 8999), 4-hydroxy-4-androstene--3,17-dione and its esters (U.S. Patent No. 4,235,893), 6-methyleneandrosta-1,4-diene-3,17-dione derivatives (Japanese Laid-open Patent Publication No. 70393/1987), 6-substituted-4-androstene-3,17-dione derivatives (Japanese Laid-open Patent Publication No. 45294/1988), 16-oxaandrosta-1,4-diene-3,17-dione (Jornal of Medicinal Chemistry 32, 651, (1989), etc.

The present inventors disclosed before that some kind of oxa- or azasteroid derivatives wherein an oxygen or nitrogen atom is introduced in the D ring portion of steroids exhibit a good aromatase inhibition action (International Publication WO 92/17489 and International Publication WO 94/07908).

The present inventors have found out that a series of steroid compounds wherein an oxygen or nitrogen atom is introduced in the D ring portion of steroids and the 6-position of the steroid skeletons is substituted with a specific substituent are slow to be inactivated through metabolism and exhibit a strong aromatase inhibition action.

### Disclosure of Invention

In the present description, the term "lower" means that the carbon atom number of a group or compound to which this term is attached is 6 or less, preferably 4 or less.

As the "lower alkyl group", there can, for example, be mentioned methyl, ethyl, n-propyl, isopropyl, n-butyl, sec-butyl, tert-butyl, n-pentyl, n-hexyl, etc.

In the above formula (I), the "acyl group" is a residue portion of an organic acid such as a mono- or polycarboxylic acid wherein at least one OH is removed, and specifically includes a group such as one represented by -COR⁴. Herein, R⁴ represents a hydrogen atom; a lower alkyl group optionally substituted with a halogen atom, an amino group, a carboxyl group, a lower alkoxycarbonyl group, a lower alkanoyloxy group, a carbamoyl group or an aryl group; a lower alkenyl group optionally substituted with an aryl group; a lower cycloalkyl group; an aryl group optionally substituted with a lower alkyl group, a lower alkoxy group or a halogen atom; an amino group optionally substituted with one or two lower alkyl groups; etc.

As specific examples of the "acyl group", there can be mentioned formyl, acetyl, propionyl, butyryl, trifluoroacetyl, glycyl, 3-carboxypropionyl, 3-ethoxycarbonylpropionyl, acetoxyacetyl, phenylacetyl, acryloyl, benzoyl, p-methoxybenzoyl, p-methylbenzoyl, p-chlorobenzoyl, carbamoyl, N,N-dimethylcarbamoyl, N,N-diethylcarbamoyl groups, etc.

In the above formula (I), a group of preferred compounds are compounds of the formula (I) wherein A represents 0 and n represents 2.

Further, another group of preferred compounds are compounds of the formula (I) wherein the bond between the 6- and 7-positions of the steroid skeleton is a single bond.

Further, another group of preferred compounds are compounds of the formula (I) wherein a double bond exists between the 1- and 2-positions of the steroid skeleton.

Further, another group of preferred compounds are compounds of the formula (I) wherein R¹ represents a lower alkylthio group, an acylthio group, a lower alkoxy group, an acyloxy group or an azido group.

When, in a compound of the formula (I) of the invention, the bond between the 6- and 7-positions of the steroid skeleton is a single bond, R¹, the substituent of the 6-position, can bind to any of the α-position and the β-position (in the chemical formulae in the present description, the substituent at the 6-position is shown by a solid line even if it can bind to any of the α-position and the β-position).

As representative examples of the compounds of the formula (I) provided by the invention, there can be mentioned the following ones besides those shown in the later-described examples.
6β-mercapto-D-homo-17-oxaandrost-4-ene-3,17a-dione,
6β-mercapto-D-homo-17-oxaandrosta-1,4-diene-3,17a-dione,
6α-(ethylthio)-D-homo-17-oxaandrosta-1,4-dien-3-one,
6β-(ethylthio)-D-homo-17-oxaandrosta-1,4-diene-3,17a-dione,
6β-(ethylthio)-D-homo-17-oxaandrost-4-en-3-one,
6β-thiocyanato-D-homo-17-oxaandrost-4-ene-3,17a-dione,
6α-(acetylthio)-D-homo-17-oxaandrost-4-ene-3,17a-dione,
6α-(propionylthio)-D-homo-17-oxaandrosta-1,4-diene-3,17a-dione,
6β-(propionylthio)-D-homo-17-oxaandrost-4-en-3-one,
6α-(benzoylthio)-D-homo-17-oxaandrosta-1,4-dien-3-one,
6β-(benzoylthio)-D-homo-17-oxaandrosta-1,4-diene-3,17a-dione,
6β-(N,N-dimethylcarbamoylthio)-D-homo-17-oxaandrosta-1,4-dien-3-one,
6β-hydroxy-D-homo-17-oxaandrost-4-en-3-one,
6β-ethoxy-D-homo-17-oxaandrost-4-ene-3,17a-dione,
6β-ethoxy-D-homo-17-oxaandrosta-1,4-diene-3,17a-dione,
6α-isopropoxy-D-homo-17-oxaandrosta-1,4-dien-3-one,
6β-isopropoxy-D-homo-17-oxaandrosta-1,4-diene-3,17a-dione,
6α-(propionyloxy)-D-homo-17-oxaandrosta-1,4-diene-3,17a-dione,
6α-(benzoyloxy)-D-homo-17-oxaandrosta-1,4-dien-3-one,
6β-azido-D-homo-17-oxaandrost-4-ene-3,17a-dione,
6α-amino-D-homo-17-oxaandrosta-1,4-diene-3,17a-dione,
6β-amino-D-homo-17-oxaandrost-4-en-3-one,
6α-mercapto-16-oxaandrosta-1,4-dien-3-one,
6β-mercapto-16-oxaandrost-4-ene-3,17-dione,
6β-(methylthio)-16-oxaandrost-4-en-3-one,
6α-thiocyanato-16-oxaandrosta-1,4-dien-3-one,
6β-thiocyanato-16-oxaandrosta-1,4-diene-3,17-dione,
6α-(acetylthio)-16-oxaandrosta-1,4-dien-3-one,
6β-(acetylthio)-16-oxaandrost-4-ene-3,17-dione,
6α-hydroxy-16-oxaandrosta-1,4-diene-3,17-dione,
6α-methoxy-16-oxaandrost-4-en-3-one,
6β-methoxy-16-oxaandrosta-1,4-diene-3,17-dione,
6β-ethoxy-16-oxaandrosta-1,4-dien-3-one,
6β-acetoxy-16-oxaandrosta-1,4-dien-3-one,
6α-azido-16-oxaandrosta-1,4-diene-3,17-dione,
6β-azido-16-oxaandrosta-1,4-dien-3-one,
6α-mercapto-16-azaandrosta-1,4-diene-3,17-dione,
6β-(methylthio)-16-azaandrosta-1,4-diene-3,17-dione,
6α-(acetylthio)-16-azaandrosta-1,4-diene-3,17-dione,
6β-(acetylthio)-16-azaandrost-4-ene-3,17-dione,
6α-(acetylthio)-17-aza-D-homoandrosta-1,4-diene-3,17a-dione,
6β-azido-17-aza-D-homoandrosta-1,4-diene-3,17a-dione.

According to the invention, a compound of the formula (I) wherein R¹ represents a group other than -O-R³ can be prepared by
(a) reacting a compound of the formula 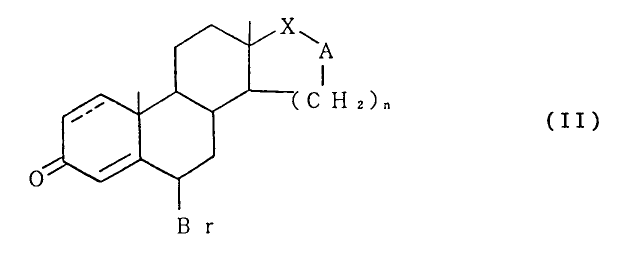 wherein X, A and n have the same meanings as defined above,
   with a compound represented by the formula

   H-R¹¹ (III)

   wherein R¹¹ represents -S-R²¹ or an azido group, and herein R²¹ represents a lower alkyl group, a cyano group or an acyl group, or a salt thereof, and subjecting the resultant compound represented by the formula 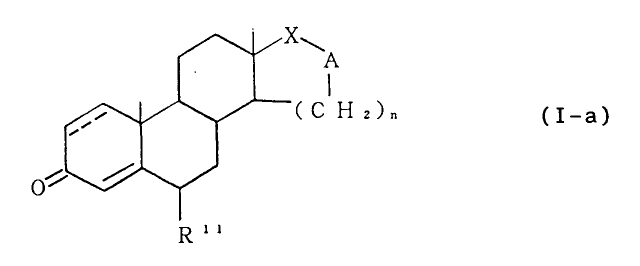 wherein R¹¹, X, A and n have the same meanings as defined above,
   if desired, to any reaction selected from
   (i) a reaction to convert the 6-position to a mercapto group,
   (ii) a reaction to convert the 6-position to a mercapto group, and then further to a lower alkylthio group or an acylthio group,
   (iii) a reaction to convert the 6-position to an amino group,
   (iv) a reaction to isomerize the 6-position, and
   (v) a reaction to introduce a double bond between the 1- and 2-positions.

   Further, according to the invention, a compound of the formula (I) wherein R¹ represents -O-R³ and the bond between the 6- and 7-positions is a single bond, can be prepared by
(b) subjecting a compound of the formula 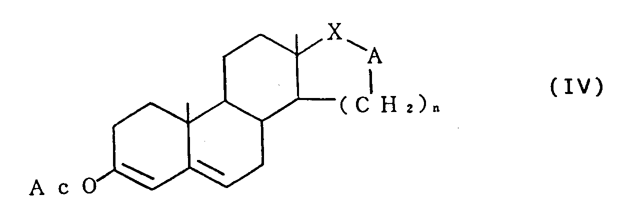 wherein Ac represents an acetyl group, and X, A and n have the same meanings as defined above,
   to oxidation reaction, and subjecting the resultant compound represented by the formula 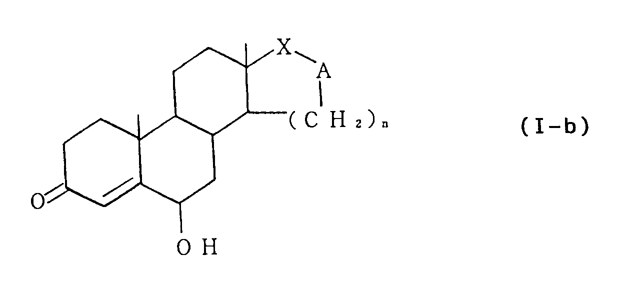 wherein X, A and n have the same meanings as defined above,
   if desired, to any reaction selected from
   (vi) a reaction to convert the 6-position to a lower alkoxy group or an acyloxy group,
      and the reactions of the above (iv) and (v).

   Further according to the invention, a compound of the formula (I) wherein R¹ represents a lower alkoxy group and there is a double bond between the 6- and 7-positions, can be prepared by
(c) treating a compound of the formula 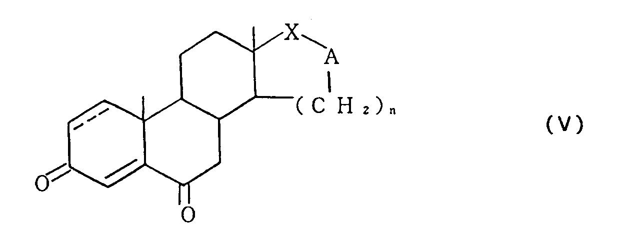 wherein X, A and n have the same meanings as defined above,
   with a lower alkanol, and subjecting the resultant compound represented by the formula 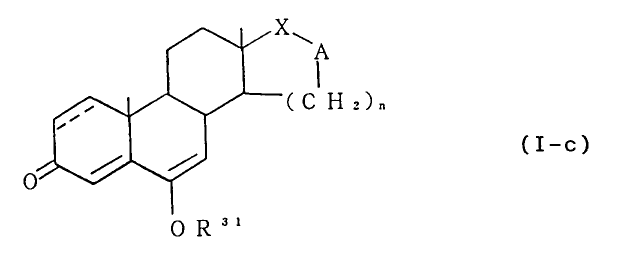 wherein R³¹ represents a lower alkyl group, and X, A and n have the same meanings as defined above,
   if desired, to the reaction of the above (v).
   According to the invention, it is also possible to prepare a compound of the above formula (I) wherein the bond between the 6- and 7-positions is a single bond, and the substituent at the 6-position is located at the β-position, by
(d) subjecting a compound of the formula 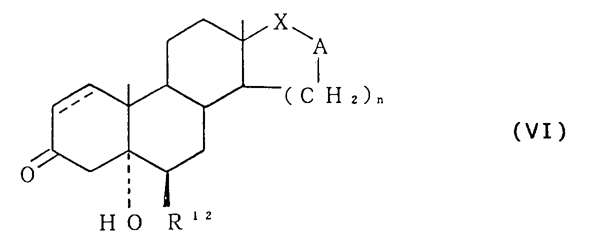 wherein R¹² represents -S-R²¹, -O-R³² or an azido group, and herein R³² represents a lower alkyl group or an acyl group, and R²¹, X, A and n have the same meanings as defined above, to dehydration reaction, and then subjecting the resultant compound of the formula 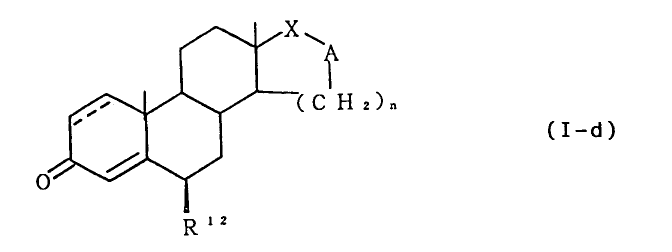 wherein R¹², X, A and n have the same meanings as defined above, if desired, to any reaction selected from

   (vii) a reaction to convert the 6-position to a hydroxyl group,
   (viii) a reaction to convert the 6-position to a hydroxyl group, and then further to a lower alkoxy group or an acyloxy group,
      and the reactions of (i) to (iii) and (v).

In the above method (a), the reaction between the compound of the formula (II) and the compound of the formula (III) can, for example, be conducted in an inert solvent such as dimethylformamide, dimethyl sulfoxide or tetrahydrofuran, in the presence of an alkali such as sodium methylate, sodium hydride, potassium butoxide or potassium hydroxide, at -20°C to the reflux temperature of the reaction mixture, preferably at a reaction temperature from room temperature to about 60°C.

The use rate of the compound of the formula (III) to the compound of the formula (II) can be at least 1 mole, preferably 1.1 to 20 moles, more preferably 1.5 to around 4 moles of the compound of the formula (III), per mole of the compound of the formula (II). Further, it is suitable to use the alkali in an amount of about 0.8 to 1.2 moles per mole of the compound of the formula (III).

When, in the above reaction, a salt, for example an alkali salt such as a potassium salt or a sodium salt of the compound of the formula (III) is used, the presence of the above alkali is unnecessary.

Thus, the compound of the formula (I-a) targeted in the invention is produced.

The obtained compound of the formula (I-a) can, if desired, be converted to another compound targeted in the invention by subjecting it to any reaction selected from the reactions of the above (i) to (v).

In the reaction of the above (i), the conversion of the 6-position to a mercapto group can readily be conducted by subjecting a compound having an acylthio group at the 6-position to hydrolysis. The hydrolysis can be conducted according to a method known per se, for example by treating the compound with an alkali such as sodium methylate, sodium hydroxide or potassium hydroxide in a solvent such as tetrahydrofuran, methanol or ethanol.

The conversion of the 6-position from the mercapto group to a lower alkylthio group in the reaction of the above (ii) can readily be conducted, for example by treatment with a lower alkylating reagent such as a lower alkyl halide, in an inert solvent such as dimethylformamide, dimethyl sulfoxide or tetrahydrofuran, in the presence of an alkali such as sodium hydride or sodium methylate. Further, the conversion of the 6-position from the mercapto group to an acylthio group can readily be conducted, for example by treatment with an acid halide, an acid anhydride or the like, in an inert solvent such as chloroform, dichloromethane or dioxane, in the presence of an alkali such as pyridine, sodium hydride or sodium methylate.

The conversion of the 6-position to an amino group in the reaction of the above (iii) can be conducted by reducing a compound having an azido group at the 6-position. The reduction can, for example, be conducted by treating the compound with triphenylphosphine and water in an inert solvent such as tetrahydrofuran.

In the reaction of the above (iv), the isomerization of the 6-position can, for example, be conducted by treatment with an acid such as hydrochloric acid, sulfuric acid or p-toluenesulfonic acid, in an inert solvent such as ethanol or chloroform.

The introduction of a double bond between the 1- and 2-positions in the reaction of the above (v) can, for example, be conducted by dehydrogenation with selenium dioxide, 2,3-dichloro-5,6-dicyano-1,4-benzoquinone (DDQ) or the like, in an inert solvent such as t-butanol, dioxane or benzene, if desired in the presence of acetic acid, pyridine or the like.

In the above process (b), the oxidation reaction of the compound of the formula (IV) can, for example, be conducted by treatment with an oxidizing agent such as, for example, m-chloroperbenzoic acid or perphthalic acid, in an inert solvent such as dioxane, ethyl ether, dichloromethane or chloroform, if desired in the presence of a phosphate buffer (pH 8), at a reaction temperature from under ice cooling to about room temperature.

Thus, the compounds of the above formula (I-b) targeted in the invention are formed.

A compound of the formula (I-b) obtained can, if desired, be converted to another compound targeted in the invention by subjecting it to any reaction selected from the reactions of the above (iv), (v) and (vi).

The conversion of the 6-position from the hydroxyl group to a lower alkoxy group in the reaction of the above (vi) can, for example, readily be conducted by treatment with a lower alkylating reagent such as a lower alkyl halide, in an inert solvent such as dimethylformamide, dimethyl sulfoxide or tetrahydrofuran, in the presence of an alkali such as sodium hydride or sodium methylate, or condensing agent such as silver oxide. Further, the conversion of the 6-position from the hydroxyl group to an acyloxy group can, for example, readily be conducted by treatment with an acid halide, an acid anhydride or the like, in an inert solvent such as chloroform, dichloromethane or dioxane, in the presence of an alkali such as pyridine, sodium hydride or sodium methylate.

A compound of the formula (I-b) can also be prepared by reducing a compound of the formula (V) with a metal hydride complex compound such as sodium borohydride or tri-t-butoxyaluminum hydride, in an inert solvent such as methanol or tetrahydrofuran, at a reaction temperature from under ice cooling to around room temperature.

When, in this reduction reaction, a compound of the formula (V) wherein X represents C = O is used, it is possible, if desired, to conduct the reduction with the group previously protected with an ethylenedioxy group or the like, and then remove the protective group after the reaction.

The treatment of the compound of the formula (V) with a lower alkanol in the above process (c) can, for example, be conducted by treating it with a lower alkanol such as methanol or ethanol, in the presence of a strong acid such as p-toluenesulfonic acid or sulfuric acid.

It is suitable that the reaction temperature is generally from under ice cooling to 80°C, preferably from room temperature to around 50°C, and it is suitable that the use rate of the strong acid is usually on the order of 10 to 20 moles per mole of the compound of the formula (V).

Thus, the compounds of the formula (I-c) targeted in the invention are formed.

The obtained compound of the formula (I-c) can, if desired, be converted to another compound targeted in the invention by subjecting it to the reaction of the above (v).

In the above process (d), the dehydration reaction of the compound of the formula (VI) can, for example, be conducted by treating it with a dehydrating agent such as thionyl chloride or phosphorus oxychloride, in a solvent such as pyridine.

It is suitable that the reaction temperature is generally from -20°C to 50°C, preferably from under ice cooling to around room temperature, and it is suitable that the use rate of the dehydrating agent is usually on the order of 2 to 10 moles per mole of the compound of the formula (VI).

Thus, the compounds of the formula (I-d) targeted in the invention is formed.

The obtained compound of the formula (I-d) can, if desired, be converted to another compound targeted in the invention by subjecting it to any of the reactions of the above (i) to (iii), (v), (vii) and (viii).

In the reaction of the above (vii), the conversion of the 6-position to a hydroxyl group can readily be conducted by subjecting a compound having an acyloxy group at the 6-position to hydrolysis. The hydrolysis can be conducted according to a process known per se, for example by treatment with an alkali such as sodium methylate, sodium hydroxide or potassium hydroxide, in a solvent such as tetrahydrofuran, methanol or ethanol.

The conversion of the 6-position from the hydroxyl group to a lower alkoxy group and the conversion thereof from the hydroxyl group to an acyloxy group in the reaction of the above (viii) can be conducted in the same manner as described in the reaction of the above (vi).

The compound of the formula (I) thus obtained can be isolated and purified from the reaction mixture according to methods known per se, for example by methods such as recrystallization, distillation, column chromatography and thin layer chromatography.

The compound of the formula (II) used as a starting material in the above process (a) is a novel compound which has not been disclosed in literatures, and can, for example, be prepared by brominating a compound of the formula 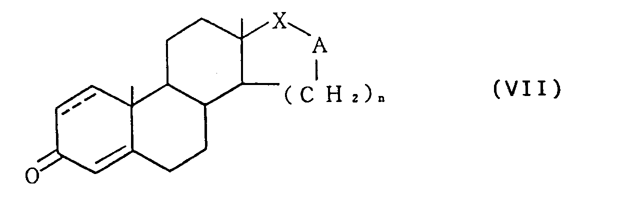 wherein X, A and n have the same meanings as defined above.

The bromination can, for example, readily be conducted by treatment with a brominating agent such as N-bromosuccinimide, in an inert solvent such as carbon tetrachloride, if desired in the presence of a catalytic amount of a radical initiator such as, for example azobisisobutyronitrile or benzoyl peroxide.

Further, the compound of the formula (IV) used as a starting material in the above process (b) is a novel compound which has not been disclosed in literatures, and can, for example, be prepared by enol acetylating a compound of the formula 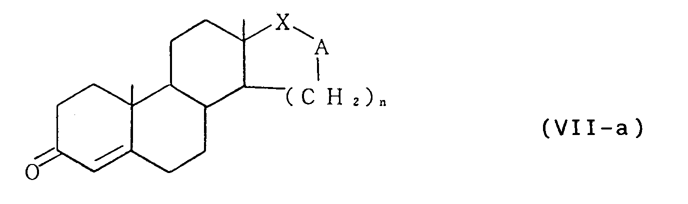 wherein X, A and n have the same meanings as defined above,
with acetic anhydride, acetyl chloride or the like, in the presence of pyridine, p-toluenesulfonic acid or the like.

In the above process (c), among compounds of the formula (V) used as a starting material , compounds having a double bond between the 1- and 2-positions are novel compounds which have not been disclosed in literatures. Such a compound can be prepared by introducing a double bond between the 1- and 2-positions of a compound of the formula (V) having a single bond between the 1-and 2-positions, in the same manner as described in the reaction of the above (v).

Further, the compounds of the formula (VI) used as a starting material in the above process (d) are also novel compounds which have not been disclosed in literatures, and can, for example, be prepared as follows. A 5-ene compound of the formula 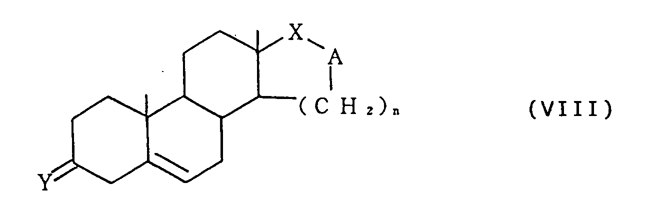 wherein Y represents a group 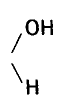 or a protected oxo group, and X, A and n have the same meanings as defined above,
is used as a starting material. When X in the formula (VIII) represents C = O, the group is, if desired, protected by an ethylenedioxy group or the like, and the resultant compound is epoxidized by treating it with m-chloroperbenzoic acid, magnesium monoperphthalate, peracetic acid or the like. The resultant 5α,6α-epoxy compound is then either treated with a compound of the formula (III) or a salt thereof to introduce a substituent R¹¹ at the 6-position, or treated with a lower alkanol in the presence of an acid such as perchloric acid or the like to introduce a lower alkoxy group at the 6-position, or treated with an acid or a reactive derivative thereof to introduce an acyloxy group at the 6-position. Thereafter, either the protective group of the oxo group is removed from the group Y, or when the group Y is a hydroxyl group, the group is converted to an oxo group through oxidation with Jones reagent or the like.

### Effect of Invention

The thus described 6-substituted oxa- or azasteroid compounds represented by the formula (I) have an excellent aromatase inhibition action, and are useful for treatment of diseases caused by estrogens, for example breast cancer, uterine cancer, ovarian cancer, endometriosis, male gynecomastia, prostatic hypertrophy, male infertility on aspermia, etc.

The results of in vitro and in vivo tests on aromatase inhibition action of the compounds of the invention are exhibited below.

### (1) Assay of aromatase inhibition action (in vitro)

Human placental microsomes (ones subjected to centrifugation at 105,000 X g for 60 minutes) were prepared according to the method of Ryan (The Journal of Biological Chemistry 234, 268-272, 1959). The microsomes were washed twice with 0.5 mM dithiothreitol solution, freeze dried, and stored at -20°C until use.

An aromatase inhibition action was assayed according to the method developed by Tompson and Siiteri (The Journal of Biological Chemistry 249, 5373-5378, 1974). Namely, the method comprises quantitatively determining ³H₂O released when [1β-³H]androstenedione is aromatized. The experiment, when the enzyme was used, was conducted in 67 mM phosphate buffer (pH 7.5) so that the amount of the final incubation solution could be 0.5 ml. The incubation solution contains 180 µM of NADPH, 2 µM of [1β-³H]androstenedione, 150 µg of freeze dried human placental microsomes, 25 µl of methanol and a test compound at various concentrations. Incubation was conducted in the air at 37°C for 20 minutes, 3 ml of chloroform was added to discontinue the reaction, and then the mixture was stirred for 40 seconds. The mixture was then centrifuged at 700 X g for 10 minutes, 0.3 ml of the aqueous solution was sampled from the supernatant, the scintillation mixture was added, and the amount of ³H₂O formed was measured.

The results are shown in the following Table 1.

**Table 1**

| Compound | IC₅₀ (µM) |
|---|---|
| Example 10 | 0.8 |
| Example 15 | 1.6 |

### (2) Assay of aromatase inhibition action (in vivo)

An aromatase inhibition action was assayed according to the method developed by G.H.Deckers and A.H.W.M.Schuurs (Journal of Steroid Biochemistry 32, 625-631, 1989).

Namely, mature female rats after one week from hypophysectomy were used with one group of 8 to 10 animals. Either 16 mg/kg of dehydroepiandrosterone sulfate (DHEAS) and 5 ml/kg of a solvent (physiological saline containing 2 % polysorbate 80), or DHEAS and a test compound suspended in the solvent were orally administered once a day for successive 4 days. For 6 days from the next day of start of the administration, the vaginal smear was sampled, and observed under a microscope after being stained with Giemsa staining solution. Cornification of the vaginal smear due to DHEAS was observed from 3 days after the initial administration, and its action disappeared 6 days thereafter. Therefore, the cornification smear number was expressed as the total of from the third day to the fifth day from the start of administration. An cornification inhibition action by the test compound was expressed as a rate to cornification in the solvent administration group.

Since cornification of the vaginal smear is caused by estrogens biosynthetically produced from DHEAS by aromatase, inhibition of the cornification is observed when aromatase is inhibited.

The results are shown in the following Table 2.

**Table 2**

| Compound | Dose (mg/kg/day) | Inhibition rate (%) |
|---|---|---|
| Example 13 (6α) | 5 | 81.3 |
| Example 15 | 2 | 90.9 |
| Example 17 | 5 | 100 |
| Example 19 | 5 | 72.3 |
| Example 31 | 5 | 100 |

The compound represented by the formula (I) of the invention can be orally or parenterally (e.g., intramuscularly, intravenously, rectally, percutaneously, etc.) administered as a biosynthesis inhibitor of estrogens for treatment of human beings or other mammals.

The compound of the invention, when used as a medicament, can be formulated into any pharmaceutical form elected from solid forms (e.g., tablets, hard capsules, soft capsules, granules, powders, fine granules, pills, troches, etc.), semisolid forms (e.g., suppositories, ointments, etc.), and liquid forms (e.g., injections, emulsions, suspensions, lotions, sprays, etc.), in accordance with its use, together with pharmaceutically acceptable non toxic additives.

As nontoxic additives usable for the formulation, there can, for example, be mentioned starches, gelatin, glucose, lactose, fructose, maltose, magnesium carbonate, talc, magnesium stearate, methylcellulose, carboxymethylcellulose or its salts, gum arabic, polyethylene glycol , alkyl p-hydroxybenzoate, syrups, ethanol, prolylene glycol, petrolatums, Carbowaxes, glycerol, sodium chloride, sodium sulfite, sodium phosphate, citric acid, etc. The medicament can also contain another therapeutically useful medicament.

The content of the compound of the invention in the medicament is varied depending on its pharmaceutical form, etc., but it is generally desirable that the medicament contains the compound of the invention at a concentration of 0.1 to 50 % by weight in the case of a solid or semi solid form, and at a concentration of 0.05 to 10 % by weight in the case of a liquid form.

The dose of the compound of the invention can widely be varied depending on the kind of mammals including human beings as a subject, administration routes, the seriousness of symptoms, the diagnoses of doctors, etc., but can generally be in the range of 0.1 to 100 mg/kg, preferably 1 to 50 mg/kg per day. However, it is of course possible to administer the compound in an amount smaller than the lower limit of the above range or in an amount larger than the upper limit thereof in accordance with seriousness of the symptom of the patient, the diagnosis of the doctor, etc. The above dose can be administered once a day or in divided several portions per day.

### Examples

The invention is further specifically described below according to examples and preparation examples.

### Example 1

(a) A mixture of 500 mg of 5,6α-epoxy-3,3-(ethylenedioxy)-D-homo-17-oxa-5α-androstane and 2 ml of thioacetic acid was stirred at room temperature for 87 hours. Water was added to the reaction mixture, and the product was extracted with ethyl acetate. The extract was washed with aqueous saturated sodium hydrogen carbonate solution and saturated saline, and dried over anhydrous sodium sulfate. The solvent was distilled off to obtain 500 mg of 6β-(acetylthio)-3,3-(ethylene-dioxy)-D-homo-17-oxa-5α-androstan-5-ol.
(b) A mixture of 500 mg of 6β-(acetylthio)-3,3-(ethylenedioxy)-D-homo-17-oxa-5α-androstan-5-ol, 8 ml of tetrahydrofuran and 1.6 ml of 3N aqueous perchloric acid was stirred at room temperature for 30 minutes. Water was added to the reaction mixture, and the product was extracted with ethyl acetate. The extract was washed with aqueous saturated sodium hydrogen carbonate solution and saturated saline, and dried over anhydrous sodium sulfate. The solvent was distilled off to obtain 548 mg of 6β-(acetylthio)-5-hydroxy-D-homo-17-oxa-5α-androstan-3-one.
(c) A mixture of 310 mg of 6β-(acetylthio)-5-hydroxy-D-homo-17-oxa-5α-androstan-3-one, 5 ml of dry pyridine and 0.6 ml of thionyl chloride was stirred under ice cooling for 3 minutes. Water was added to the reaction mixture, and the product was extracted with ethyl acetate. The extract was washed with diluted hydrochloric acid and saturated saline, and dried over anhydrous sodium sulfate. The solvent was distilled off to obtain 272 mg of 6β-(acetylthio)-D-homo-17-oxaandrost-4-en-3-one.
   ¹H-NMR (CDCl₃,δ): 1.04 (3H,s), 1.28 (3H,s), 2.32 (3H,s), 2.97, 3.42 (2H,ABq,J=10.8Hz), 3.2-3.5 (1H,m), 3.9-4.2 (1H,m), 4.5-4.7 (1H,m), 5.95 (1H,s)
   MS (m/z): 362 (M⁺), 320, 287

### Example 2

A mixture of 15 mg of 6β-(acetylthio)-D-homo-17-oxaandrost-4-en-3-one, 2.5 ml of ethanol and 0.25 ml of 1N hydrochloric acid was refluxed for 3 hours. The reaction mixture was diluted with ethyl acetate, washed with aqueous saturated sodium hydrogen carbonate solution and saturated saline, and dried over anhydrous sodium sulfate. The solvent was distilled off, and the resultant crude product was purified by TLC [developing solvent, chloroform : ethyl acetate (40 : 1)] to obtain 3 mg of 6α-(acetylthio)-D-homo-17-oxaandrost-4-en-3-one.
¹H-NMR (CDCl₃,δ): 1.04 (3H,s), 1.30 (3H,s), 2.35 (3H,s), 2.96, 3.41 (2H,ABq,J=11Hz), 3.2-3.4 (1H,m), 3.9-4.2 (1H,m), 4.33 (1H,ddd,J=2, 5.1, 13.2Hz), 6.08 (1H,d,J=2Hz)
MS (m/z): 362 (M⁺), 320, 287

### Example 3

A mixture of 380 mg of 6β-(acetylthio)-D-homo-17-oxaandrost-4-en-3-one, 50 ml of tertiary butanol, 2.2 ml of acetic acid and 473 mg of selenium dioxide was refluxed for 20 hours. The reaction mixture was left alone to cool , water was added thereto, and the product was extracted with ethyl acetate. The extract was washed with cold 1N solution of sodium hydroxide and cold diluted sulfuric acid, and dried over anhydrous sodium sulfate. The solvent was distilled off, and the resultant crude product was purified by TLC [developing solvent, chloroform : ethyl acetate (40 : 1)] to obtain 155 mg of 6β-(acetylthio)-D-homo-17-oxaandrosta-1,4-dien-3-one.
¹H-NMR (CDCl₃,δ): 1.07 (3H,s), 1.32 (3H,s), 2.34 (3H,s), 2.97, 3.41 (2H,ABq,J=11Hz), 3.2-3.5 (1H,m), 3.9-4.2 (1H,m), 4.6-4.7 (1H,m), 6.1-6.3 (2H,m), 7.01 (1H,d,J=10.1Hz)
MS (m/z): 360 (M⁺), 318, 284

### Example 4

A mixture of 50 mg of 6β-(acetylthio)-D-homo-17-oxaandrosta-1,4-dien-3-one, 0.2 ml of methanol and 0.3 ml of 3.5 % solution of sodium methylate in methanol was stirred in a stream of nitrogen at 0°C for 30 minutes. 2 ml of 1N hydrochloric acid was added to the reaction mixture, and the resultant precipitate was taken by filtration. The obtained crude product was purified by TLC [developing solvent, benzene : ethyl acetate (4 : 1)] to obtain 7 mg of 6β-mercapto-D-homo-17-oxaandrosta-1,4-dien-3-one.
¹H-NMR (CDCl₃,δ): 1.10 (3H,s), 1 .55 (3H,s), 2.97, 3.43 (2H,ABq,J=11Hz), 3.2-3.4 (1H,m), 3.9-4.3 (2H,m), 6.1-6.3 (2H,m), 7.02 (1H,d,J=10.1Hz)
MS (m/z): 318 (M⁺), 284

### Example 5

A mixture of 0.12 ml of thioacetic acid, 0.28 ml of 28 % solution of sodium methylate in methanol and 4 ml of dimethylformamide was stirred in an atmosphere of nitrogen at room temperature for 30 minutes. 420 mg of 6ξ-bromo-D-homo-17-oxaandrost-4-en-3-one was added to this mixture, and the mixture was stirred at the same temperature for one hour. Water was added to the reaction mixture, and the product was extracted with ethyl acetate. The extract was washed with aqueous saturated sodium hydrogen carbonate solution and saturated saline, and dried over anhydrous magnesium sulfate. The solvent was distilled off, and the resultant crude product was purified by TLC [developing solvent, chloroform : ethyl acetate (40 : 1)] to obtain 67 mg of 6α-(acetylthio)-D-homo-17-oxaandrost-4-en-3-one as a low polar compound.

Further, 95 mg of 6β-(acetylthio)-D-homo-17-oxaandrost-4-en-3-one was obtained as a high polar compound.

### Example 6

(a) A mixture of 500 mg of 5,6α-epoxy-3,3-(ethylenedioxy)-D-homo-17-oxa-5α-androstane, 2.4 g of potassium thiocyanate and 150 ml of acetic acid was stirred at 80°C for one hour. The reaction mixture was concentrated under reduced pressure, cold saturated solution of sodium carbonate was added, and the product was extracted with methylene chloride. The extract was washed with water, and dried over anhydrous sodium sulfate. The solvent was distilled off to obtain 622 mg of 3,3-(ethylenedioxy)-6β-thiocyanato-D-homo-17-oxa-5α-androstan-5-ol.
(b) The same procedure as in (b) and (c) of Example 1 was conducted except that 622 mg of 3,3-(ethylenedioxy)-6β-thiocyanato-D-homo-17-oxa-5α-androstan-5-ol was used in place of 6β-(acetylthio)-3,3-(ethylenedioxy)-D-homo-17-oxa-5α-androstan-5-ol, and the resultant crude product was purified by TLC [developing solvent, chloroform : ethyl acetate (40 : 1)] to obtain 90 mg of 6β-thiocyanato-D-homo-17-oxaandrost-4-en-3-one.
   ¹H-NMR (CDCl₃,δ): 1.06 (3H,s), 1.43 (3H,s), 2.98, 3.43 (2H,ABq, J=11Hz), 3.2-3.4 (1H,m), 3.9-4.2 (1H,m), 4.4-4.5 (1H,m), 5.92 (1H,s)
   MS (m/z): 345 (M⁺)

### Example 7

(a) A mixture of 50 mg of 5,6α-epoxy-3,3-(ethylenedioxy)-D-homo-17-oxa-5α-androstane, 3.2 ml of 2-methoxyethanol-water (3 : 1) and 289 mg of sodium azide was stirred in an atmosphere of nitrogen at 85°C for 63 hours. The reaction mixture was poured into ice water, and the product was extracted with methylene chloride. The extract was washed with water, and dried over anhydrous sodium sulfate. The solvent was distilled off, and the resultant crude product was purified by TLC [developing solvent, chloroform : ethyl acetate (40 : 1)] to obtain 49 mg of 6β-azido-3,3-(ethylenedioxy)-D-homo-17-oxa-5α-androstan-5-ol.
   ¹H-NMR (CDCl₃,δ): 0.99 (3H,s), 1.08 (3H,s), 2.98, 3.38 (2H,ABq, J=11Hz), 3.3-3.5 (2H,m), 3.8-4.2 (5H,m), 4.36(1H,s)
(b) The same procedure as in (b) and (c) of Example 1 was conducted except that 49 mg of 6β-azido-3,3-(ethylenedioxy)-D-homo-17-oxa-5α-androstan-5-ol was used in place of 6β-(acetylthio)-3,3-(ethylenedioxy)-D-homo-17-oxa-5α-androstan-5-ol , and the resultant crude product was purified by TLC [developing solvent, chloroform : ethyl acetate (40 : 1)] to obtain 9 mg of 6β-azido-D-homo-17-oxaandrost-4-en-3-one.
   ¹H-NMR (CDCl₃,δ): 1.04 (3H,s), 1.37 (3H,s), 2.97, 3.42 (2H,ABq, J=11Hz), 3.2-3.5 (1H,m), 3.9-4.3 (2H,m), 5.81 (1H,s)
   MS (m/z): 329 (M⁺), 301, 287

### Example 8

The same procedure as in Example 3 was conducted except that 196 mg of 6β-azido-D-homo-17-oxaandrost-4-en-3-one was used in place of 6β-(acetylthio)-D-homo-17-oxaandrost-4-en-3-one, whereby 39 mg of 6β-azido-D-homo-17-oxaandrosta-1,4-dien-3-one was obtained.
¹H-NMR (CDCl₃,δ): 1.07 (3H,s), 1.44 (3H,s), 2.96, 3.42 (2H,ABq,J=11Hz), 3.1-3.4 (1H,m), 3.9-4.2 (1H,m), 4.3-4.4 (1H,m), 6.1-6.3 (2H,m), 7.05 (1H,d,J=11Hz)
MS (m/z): 299, 284, 270

### Example 9

(a) A mixture of 500 mg of 5,6α-epoxy-3,3-(ethylenedioxy)-D-homo-17-oxa-5α-androstane, 2 ml of aqueous 15 % sodium methanethiolate solution and 4 ml of dimethylformamide was stirred at 80°C for 20 hours. Water was added to the reaction mixture, and the product was extracted with ethyl acetate. The extract was washed with aqueous 5 % sodium hydroxide solution and saturated saline, and dried over anhydrous magnesium sulfate. The solvent was distilled off to obtain 516 mg of 3,3-(ethylenedioxy)-6β-(methylthio)-D-homo-17-oxa-5α-androstan-5-ol.
(b) The same procedure as in (b) and (c) of Example 1 was conducted except that 516 mg of 3,3-(ethylenedioxy)-6β-(methylthio)-D-homo-17-oxa-5α-androstan-5-ol was used in place of 6β-(acetylthio)-3,3-(ethylenedioxy)-D-homo-17-oxa-5α-androstan-5-ol, and the resultant crude product was purified by TLC [developing solvent, chloroform : ethyl acetate (40 : 1)] to obtain 270 mg of 6β-(methylthio)-D-homo-17-oxaandrost-4-en-3-one.
   ¹H-NMR (CDCl₃,δ): 1.04 (3H,s), 1.45 (3H,s), 2.00 (3H,s), 2.98, 3.42 (2H,ABq,J=11Hz), 3.2-3.5 (2H,m), 3.9-4.2 (1H,m), 5.63 (1H,s)

### Example 10

The same procedure as in Example 3 was conducted except that 194 mg of 6β-(methylthio)-D-homo-17-oxaandrost-4-en-3-one was used in place of 6β-(acetylthio)-D-homo-17-oxaandrost-4-en-3-one, whereby 46 mg of 6β-(methylthio)-D-homo-17-oxaandrosta-1,4-dien-3-one was obtained.
¹H-NMR (CDCl₃,δ): 1.08 (3H,s), 1.53 (3H,s), 2.05 (3H,s), 2.97, 3.42 (2H,ABq,J=11Hz), 3.2-3.4 (1H,m), 3.6-3.7 (1H,m), 3.9-4.2 (1H,m), 6.07 (1H,d,J=1.8Hz), 6.22 (1H,dd,J=1.8, 10Hz), 7.03 (1H,d,J=10Hz)
MS (m/z): 332 (M⁺), 284

### Example 11

The same procedure as in (c) of Example 1 was conducted except that 320 mg of 6β-(acetylthio)-5-hydroxy-D-homo-17-oxa-5α-androstane-3,17a-dione was used in place of 6β-(acetylthio)-5-hydroxy-D-homo-17-oxa-5α-androstan-3-one, and the resultant crude product was purified by TLC [developing solvent, chloroform : ethyl acetate (19 : 1)] to obtain 78 mg of 6β-(acetylthio)-D-homo-17-oxaandrost-4-en-3,17α-dione.
¹H-NMR (CDCl₃,δ): 1.28 (6H,s), 2.33 (3H,s), 4.2-4.5 (2H,m), 4.5-4.7 (1H,m), 5.96 (1H,s)
MS (m/z): 376 (M⁺), 334, 301

### Example 12

To a mixture of 20 mg of 6β-bromo-D-homo-17-oxaandrosta-1,4-diene-3,17a-dione and 1.8 ml of dimethylformamide was added 0.1 ml of aqueous 8.3 % sodium azide solution, and the mixture was stirred in an atmosphere of nitrogen at 100°C for 2 hours. The reaction mixture was left alone to cool, diluted hydrochloric acid was added thereto, and the product was extracted with ethyl acetate. The extract was washed with saturated saline, and dried over anhydrous magnesium sulfate. The solvent was distilled off, and the resultant crude product was purified by TLC [developing solvent, hexane : ethyl acetate (1 : 1)] to obtain 5 mg of 6α-azido-D-homo-17-oxaandrosta-1,4-diene-3,17a-dione.
¹H-NMR (CDCl₃,δ): 1.25 (3H,s), 1.30 (3H,s), 4.0-4.7 (3H,m), 6.29 (1H,dd,J=2, 10Hz), 6.43 (1H,m), 7.01 (1H,d,J=10Hz)
MS (m/z): 342 (MH⁺), 327, 313, 298

### Example 13

A mixture of 50 mg of 6β-bromo-D-homo-17-oxaandrosta-1,4-diene-3,17a-dione, 43 mg of potassium thiocyanate and 2 ml of dimethylformamide was stirred in an atmosphere of nitrogen at 60°C. Seven hours later, the same amount of potassium thiocyanate was added, and the mixture was stirred under the same condition for further 17 hours. The reaction mixture was left alone to cool, diluted hydrochloric acid was added thereto, and the product was extracted with ethyl acetate. The extract was washed with aqueous 5 % sodium hydrogen carbonate solution and saturated saline, and dried over anhydrous magnesium sulfate. The solvent was distilled off, and the resultant crude product was purified by TLC [once by developing solvent, hexane : ethyl acetate (1 : 1), and then twice by developing solvent, chloroform : acetone (19 : 1)] to obtain 12 mg of 6β-thiocyanato-D-homo-17-oxaandrosta-1,4-diene-3,17a-dione as a low polar compound.
IR (KBr,v,cm⁻¹): 3460, 2952, 2072, 1732
¹H-NMR (CDCl₃,δ): 1.35 (3H,s), 1.46 (3H,s), 4.1-4.7 (2H,m), 4.76 (1H,m), 6.21-6.34 (2H,m), 7.03 (1H,d,J=10Hz)
MS (m/z): 357 (M⁺), 342, 299

Further, 6 mg of 6α-thiocyanato-D-homo-17-oxaandrosta-1,4-diene-3,17a-dione was obtained as a high polar compound.
IR (KBr,v,cm⁻¹): 3464, 2952, 2156, 1732
¹H-NMR (CDCl₃,δ): 1.30 (6H,s), 2.5-2.8 (1H,m), 4.1-4.7 (3H,m), 6.25-6.43 (2H,m), 7.05 (1H,d,J=10Hz)
MS (m/z): 357 (M⁺), 342

### Example 14

The same procedure as in Example 13 was conducted except that 140 µl of aqueous 15 % sodium methanethiolate solution was used in place of potassium thiocyanate. The resultant crude product was purified by TLC [developing solvent, hexane : ethyl acetate (1 : 1)] and then purified by TLC [developing solvent, chloroform : acetone (39 : 1)] to obtain 4 mg of 6β-(methylthio)-D-homo-17-oxaandrosta-1,4-diene-3,17a-dione.
¹H-NMR (CDCl₃,δ): 1.32 (3H,s), 1.53 (3H,s), 2.07 (3H,s), 3.68 (1H,dd,J=1.1, 5.5Hz), 4.1-4.5 (2H,m), 6.08 (1H,d,J=2Hz), 6.24 (1H,dd,J=2, 10Hz), 7.03 (1H,d,J=10Hz)
MS (m/z): 346 (M⁺), 331, 299, 298

### Example 15

The same procedure as in Example 5 was conducted except that 130 mg of 6β-bromo-D-homo-17-oxaandrosta-1,4-diene-3,17a-dione was used in place of 6ξ-bromo-D-homo-17-oxaandrost-4-en-3-one, and the resultant crude product was purified by TLC [developing solvent, chloroform : acetone (19 : 1)] to obtain 78 mg of 6α-(acetylthio)-D-homo-17-oxaandrosta-1,4-diene-3,17a-dione.
¹H-NMR (CDCl₃,δ): 1.31 (3H,s), 1.37 (3H,s), 2.38 (3H,s), 4.0-4.6 (3H,m), 6.20-6.39 (2H,m), 7.05 (1H,d,J=10Hz)
MS (m/z): 374 (M⁺), 359, 332, 288

### Example 16

A mixture of 20 mg of 6α-(acetylthio)-D-homo-17-oxaandrosta-1,4-diene-3,17a-dione, 11 µl of 28 % solution of sodium methylate in methanol and 0.1 ml of methanol was stirred in an atmosphere of nitrogen at 0°C for 30 minutes. Diluted hydrochloric acid was added to the reaction mixture, and the product was extracted with ethyl acetate. The extract was washed with saturated saline, and dried over anhydrous magnesium sulfate. The solvent was distilled off, and the resultant crude product was purified by TLC [developing solvent, chloroform : acetone (19 : 1)] to obtain 3 mg of 6α-mercapto-D-homo-17-oxaandrosta-1,4-diene-3,17a-dione.
¹H-NMR (CDCl₃,δ): 1.26 (3H,s), 1.29 (3H,s), 3.5-4.0 (1H,m), 4.0-4.6 (2H,m), 6.30 (1H,dd,J=2, 10Hz), 6.71-6.74 (1H,m), 7.03 (1H,d,J=10Hz)
MS (m/z): 332 (M⁺), 317, 298

### Example 17

A mixture of 50 mg of 6β-bromo-D-homo-17-oxaandrosta-1,4-dien-3-one, 18 mg of sodium azide and 1 ml of dimethylformamide was stirred in an atmosphere of nitrogen at room temperature for 2 hours. Water was added to the reaction mixture, and the product was extracted with ethyl acetate. The extract was washed with saturated saline, and dried over anhydrous magnesium sulfate. The solvent was distilled off, and the resultant crude product was purified by TLC [developing solvent, hexane : ethyl acetate (1 : 1)], and further purified by TLC [developing solvent, benzene : ethyl acetate (9 : 1)] to obtain 22 mg of 6α-azido-D-homo-17-oxaandrosta-1,4-dien-3-one.
¹H-NMR (CDCl₃,δ): 1.06 (3H,s), 1.25 (3H,s), 2.26-2.49 (1H,m), 2.97, 3.42 (2H,ABq,J=11Hz), 3.2-3.5 (1H,m), 3.9-4.4 (2H,m), 6.27 (1H,dd,J=1.5, 10Hz), 6.41 (1H,br s), 7.01 (1H,d,J=10Hz)
MS (m/z): 328 (MH⁺), 299, 284

### Example 18

The same procedure as in Example 17 was conducted for 16 hours except that 175 mg of potassium thiocyanate was used in place of sodium azide, and the resultant crude product was purified by TLC [developing solvent, chloroform : acetone (19 : 1)] to obtain 18 mg of 6β-thiocyanato-D-homo-17-oxaandrosta-1,4-dien-3-one as a low polar compound.
¹H-NMR (CDCl₃,δ): 1.11 (3H,s), 1.46 (3H,s), 2.97, 3.43 (2H,ABq, J=11Hz), 3.2-3.5 (1H,m), 3.9-4.2 (1H,m), 4.69-4.76 (1H,m), 6.19-6.32 (2H,m), 7.04 (1H,d,J=10Hz)
MS (m/z): 343 (M⁺), 328, 284

Further, 8 mg of 6α-thiocyanato-D-homo-17-oxaandrosta-1,4-dien-3-one was obtained as a high polar compound.
¹H-NMR (CDCl₃,δ): 1.06 (3H,s), 1.30 (3H,s), 2.44-2.66 (1H,m), 2.97, 3.43 (2H,ABq, J=11Hz), 3.2-3.5 (1H,m), 3.9-4.3 (2H,m), 6.31 (1H,dd,J=1.8, 10Hz), 6.41-6.45 (1H,m), 7.05 (1H,d,J=10Hz)
MS (m/z): 343 (M⁺), 328, 285

### Example 19

The same procedure as in Example 5 was conducted except that 700 mg of 6β-bromo-D-homo-17-oxaandrosta-1,4-dien-3-one was used in place of 6ξ-bromo-D-homo-17-oxaandrost-4-en-3-one. The resultant crude product was purified by TLC [developing solvent, chloroform : acetone (39 : 1)], and then purified by TLC [developing solvent, benzene : ethyl acetate (4 : 1)] to obtain 370 mg of 6α-(acetylthio)-D-homo-17-oxaandrosta-1,4-dien-3-one.
¹H-NMR (CDCl₃,δ): 1.06 (3H,s), 1.37 (3H,s), 2.36 (3H,s), 2.94, 3.40 (2H,ABq ,J=11Hz), 3.1-3.4 (1H,m), 3.9-4.2 (1H,m), 4.42 (1H,ddd,J=1.5, 4.6, 13Hz), 6.25 (1H,dd,J=2, 10Hz), 6.36-6.39 (1H,m), 7.05 (1H,d,J=10Hz)
MS (m/z): 360 (M⁺), 345, 318, 284

### Example 20

The same procedure as in Example 16 was conducted except that 25 mg of 6α-(acetylthio)-D-homo-17-oxaandrosta-1,4-dien-3-one was used in place of 6α-(acetylthio)-D-homo-17-oxaandrosta-1,4-diene-3,17a-dione, whereby 3 mg of 6α-mercapto-D-homo-17-oxaandrosta-1,4-dien-3-one was obtained.
¹H-NMR (CDCl₃,δ): 1.05 (3H,s), 1.26 (3H,s), 2.31-2.53 (1H,m), 2.90, 3.41 (2H,ABq,J=11Hz), 3.2-3.4 (1H,m), 3.6-4.2 (2H,m), 6.27 (1H,dd,J=2, 10Hz), 6.69-6.73 (1H,m), 7.03 (1H,d,J=10Hz)
MS (m/z): 318 (M⁺), 303, 284

### Example 21

A mixture of 5 mg of 6α-mercapto-D-homo-17-oxaandrosta-1,4-dien-3-one, 20 µℓ of propionic anhydride and 0.25 ml of pyridine was stirred at room temperature for 16 hours. Diluted hydrochloric acid was added to the reaction mixture, and the product was extracted with ethyl acetate. The extract was washed with aqueous 5 % sodium hydrogen carbonate solution and saturated saline, and dried over anhydrous magnesium sulfate. The solvent was distilled off, and the resultant crude product was purified by TLC [developing solvent, hexane : ethyl acetate (1 : 1)] to obtain 2 mg of 6α-(propionylthio)-D-homo-17-oxaandrosta-1,4-dien-3-one.
1H-NMR (CDCl₃,δ): 1.06 (3H,s), 1.19 (3H,t,J=7Hz), 1.38 (3H,s), 2.61 (2H,q,J=7Hz), 2.95, 3.41 (2H,ABq,J=11Hz), 3.1-3.6 (1H,m), 3.9-4.2 (1H,m), 4.43 (1H,ddd,J=1.5, 4.6, 13Hz), 6.26 (1H,dd,J=2, 10Hz), 6.39 (1H,m), 7.05 (1H,d,J=10Hz)
MS (m/z): 374 (M⁺), 359, 341, 318, 284

### Example 22

A mixture of 20 mg of 6α-azido-D-homo-17-oxaandrosta-1,4-dien-3-one, 26 mg of triphenylphosphine and 0.1 ml of tetrahydrofuran was stirred at room temperature for 2 hours. 33 µl of water was added to the reaction mixture, and the mixture was refluxed for 5 minutes. Diluted hydrochloric acid was added to the reaction mixture, and the water layer was washed with methylene chloride. The water layer was made to pH 10 with aqueous 10 % sodium hydroxide solution, the product was extracted with methylene chloride, and the extract was dried over anhydrous magnesium sulfate. The solvent was distilled off, and the resultant crude product was purified by TLC [developing solvent, chloroform : methanol (9 : 1)] to obtain 7 mg of 6α-amino-D-homo-17-oxaandrosta-1,4-dien-3-one.
¹H-NMR (CDCl₃,δ): 1.05 (3H,s), 1.25 (3H,s), 2.1-2.4 (1H,m), 2.5-2.7 (2H,br), 2.95, 3.41 (2H,ABq,J= 11Hz), 3.1-3.5 (1H,m), 3.76 (1H,br dd ,J=5, 12Hz), 3.9-4.2 (1H,m), 6.26 (1H,dd,J=1.8, 10Hz), 6.39 (1H,m), 7.04 (1H,d,J=10Hz)
MS (m/z): 301 (M⁺), 284

### Example 23

51 mg of 2α-bromo-5-hydroxy-6β-methoxy-D-homo-17-oxa-5α-androstan-3-one was added to a refluxing mixture of 0.7 ml of dimethylformamide and 40 mg of calcium carbonate, and the mixture was refluxed for 15 minutes. The reaction mixture was left alone to cool, diluted with diethyl ether, washed with 10 % hydrochloric acid, aqueous saturated sodium carbonate solution and saturated saline, and dried over anhydrous sodium sulfate. The solvent was distilled off, and the resultant crude product was purified by TLC [developing solvent, chloroform : acetone (19 : 1)] to obtain 6 mg of 5-hydroxy-6β-methoxy-D-homo-17-oxa-5α-androst-1-en-3-one as a high polar compound.
¹H-NMR (CDCl₃,δ): 1.03 (3H,s), 1.24 (3H,s), 2.9-3.6 (4H,m), 3.32 (3H,s), 3.9-4.2 (1H,m), 5.95 (1H,dd,J=1, 10Hz), 6.99 (1H,d,J=10Hz) MS (m/z): 334 (M⁺), 316
Further, 6 mg of 6β-methoxy-D-homo-17-oxaandrosta-1,4-dien-3-one as a low polar compound.
¹H-NMR (CDCl₃,δ): 1.07 (3H,s), 1.38 (3H,s), 2.96, 3.41 (2H,ABq, J=11Hz), 3.24 (3H,s), 3.3-3.5 (1H,m), 3.88 (1H,t,J=3Hz), 3.9-4.2 (1H,m), 6.2-6.3 (2H,m), 7.05 (1H,d,J=11Hz)
MS (m/z): 316 (M⁺), 301

### Example 24

The same procedure as in Example 1 (c) was conducted except that 6 mg of 5-hydroxy-6β-methoxy-D-homo-17-oxa-5α-androst-1-en-3-one was used in place of 6β-(acetylthio)-5-hydroxy-D-homo-17-oxa-5α-androstan-3-one, whereby 3 mg of 6β-methoxy-D-homo-17-oxaandrosta-1,4-dien-3-one was obtained.

### Example 25

The same procedure as in Example 1 (c) was conducted except that 823 mg of 5-hydroxy-6β-methoxy-D-homo-17-oxa-5α-androstan-3-one was used in place of 6β-(acetylthio)-5-hydroxy-D-homo-17-oxa-5α-androstan-3-one, whereby 404 mg of 6β-methoxy-D-homo-17-oxaandrost-4-en-3-one was obtained.
¹H-NMR (CDCl₃,δ): 1.04 (3H,s), 1.28 (3H,s), 2.98, 3.41 (2H,ABq, J=11Hz), 3.18 (3H,s), 3.3-3.5 (1H,m), 3.67 (1H,t,J=3Hz), 4.0-4.2 (1H,m), 5.79 (1H,s) MS (m/z): 318 (M⁺), 303

### Example 26

The same procedure as in Example 3 was conducted except that 390 mg of 6β-methoxy-D-homo-17-oxaandrost-4-en-3-one was used in place of 6β-(acetylthio)-D-homo-17-oxaandrost-4-en-3-one, and the resultant crude product was purified by TLC [developing solvent, chloroform : acetone (19 : 1)] to obtain 160 mg of 6β-methoxy-D-homo-17-oxaandrosta-1,4-dien-3-one.

### Example 27

(a) A mixture of 1 g of 5,6α-epoxy-3,3-(ethylenedioxy)-D-homo-17-oxa-5α-androstane and 40 ml of acetic acid was stirred at 100°C for one hour. The reaction mixture was left alone to cool, water was added thereto, and the product was extracted with diethyl ether. The extract was washed with 1 N aqueous sodium hydroxide solution and saturated saline, and dried over anhydrous sodium sulfate. The solvent was distilled off to obtain 1016 mg of 6β-acetoxy-3,3-(ethylenedioxy)-D-homo-17-oxa-5α-androstan-5-ol.
(b) The same procedure as in Example 1 (b) was conducted except that 1 g of 6β-acetoxy-3,3-(ethylenedioxy)-D-homo-17-oxa-5α-androstan-5-ol was used in place of 6β-(acetylthio)-3,3-(ethylenedioxy)-D-homo-17-oxa-5α-androstan-5-ol, whereby 1 g of 6β-acetoxy-5-hydroxy-D-homo-17-oxa-5α-androstan-3-one was obtained.
   ¹H-NMR (CDCl₃,δ): 1.02 (3H,s), 1.31 (3H,s), 2.08 (3H,s), 2.98, 3.40 (2H,ABq,J=11Hz), 3.3-3.5 (1H,m), 3.9-4.2 (1H,m), 4.71 (1H,br s)
   MS (m/z): 364 (M⁺), 346
(c) The same procedure as in Example 1 (c) was conducted except that 894 mg of 6β-acetoxy-5-hydroxy-D-homo-17-oxa-5α-androstan-3-one was used in place of 6β-(acetylthio)-5-hydroxy-D-homo-17-oxa-5α-androstan-3-one, whereby 936 mg of 6β-acetoxy-D-homo-17-oxaandrost-4-en-3-one was obtained.
   ¹H-NMR (CDCl₃,δ): 1.06 (3H,s), 1.29 (3H,s), 2.04 (3H,s), 2.99, 3.42 (2H,ABq,J=11Hz), 3.3-3.5 (1H,m), 4.0-4.2 (1H,m), 5.45 (1H, t,J=3Hz), 5.95 (1H,s)
   MS (m/z): 346 (M⁺), 304

### Example 28

The same procedure as in Example 3 was conducted except that 450 mg of 6β-acetoxy-D-homo-17-oxaandrost-4-en-3-one was used in place of 6β-(acetylthio)-D-homo-17-oxaandrost-4-en-3-one, and the resultant crude product was purified by TLC [developing solvent, chloroform : acetone (19 : 1)] to obtain 102 mg of 6β-acetoxy-D-homo-17-oxaandrosta-1,4-dien-3-one.
¹H-NMR (CDCl₃,δ): 1.10 (3H,s), 1.34 (3H,s), 2.06 (3H,s), 2.98, 3.43 (2H,ABq ,J=11Hz), 3.3-3.5 (1H,m), 3.9-4.2 (1H,m), 5.51 (1H,t,J=3Hz), 6.2-6.3 (2H,m), 7.02 (1H,d,J=10Hz)
MS (m/z): 345 (MH⁺), 328, 302, 284

### Example 29

(a) To a mixture of 500 mg of 5,6α-epoxy-3,3-(ethylenedioxy)-D-homo-17-oxa-5α-androstane, 3.2 ml of tetrahydrofuran and 2.5 ml of ethanol was added, under ice cooling, 3.1 ml of 3 N aqueous perchloric acid solution, and the mixture was stirred for 15 minutes. Water was added to the reaction mixture, and the product was extracted with ethyl acetate. The extract was washed with aqueous saturated sodium hydrogen carbonate solution and saturated saline, and dried over anhydrous sodium sulfate. The solvent was distilled off to obtain 495 mg of 6β-ethoxy-5-hydroxy-D-homo-17-oxa-5α-androstan-3-one.
(b) The same procedure as in Example 1 (c) was conducted except that 490 mg of 6β-ethoxy-5-hydroxy-D-homo-17-oxa-5α-androstan-3-one was used in place of 6β-(acetylthio)-5-hydroxy-D-homo-17-oxa-5α-androstan-3-one, whereby 274 mg of 6β-ethoxy-D-homo-17-oxaandrost-4-en-3-one was obtained.
   ¹H-NMR (CDCl₃,δ): 1.05 (3H,s), 1.14 (3H,t,J=7Hz), 1.28 (3H,s), 2.9-3.6 (5H,m), 3.78 (1H,t,J=3Hz), 4.0-4.2 (1H,m), 5.76 (1H,s)
   MS (m/z): 332 (M⁺), 317

### Example 30

The same procedure as in Example 3 was conducted except that 250 mg of 6β-ethoxy-D-homo-17-oxaandrost-4-en-3-one was used in place of 6β-(acetylthio)-D-homo-17-oxaandrost-4-en-3-one, and the resultant crude product was purified by TLC [developing solvent, chloroform : ace tone (19 : 1)] to obtain 106 mg of 6β-ethoxy-D-homo-17-oxaandrosta-1,4-dien-3-one.
¹H-NMR (CDCl₃,δ): 1.08 (3H,s), 1.17 (3H,t,J=7Hz), 1.38 (3H,s), 2.9-3.6 (5H,m), 3.9-4.2 (2H,m), 6.1-6.4 (2H,m), 7.05 (1H,d,J=10Hz)
MS (m/z): 330 (M⁺), 315, 301

### Example 31

The same procedure as in Example 23 was conducted except that 49 mg of 2α-bromo-5-hydroxy-6β-methoxy-D-homo-17-oxa-5α- androstane-3,17a-dione was used in place of 2α-bromo-5-hydroxy-6β-methoxy-D-homo-17-oxa-5α-androstan-3-one, and the resultant crude product was purified by TLC [developing solvent, chloroform : acetone (9 : 1)] to obtain 1 mg of 6β-methoxy-D-homo-17-oxaandrosta-1,4-diene-3,17a-dione.
¹H-NMR (CDCl₃,δ): 1.31 (3H,s), 1.37 (3H,s), 3.25 (3H,s), 3.8-4.0 (1H,m), 4.1-4.6 (2H,m), 6.2-6.4 (2H,m), 7.05 (1H,d,J=10Hz)
MS (m/z): 330 (M⁺), 315

### Example 32

The same procedure as in Example 1 (c) was conducted except that 530 mg of 5-hydroxy-6β-methoxy-D-homo-17-oxa-5α-androstane-3,17a-dione was used in place of 6β-(acetylthio)-5-hydroxy-D-homo-17-oxa-5α-androstan-3-one, whereby 230 mg of 6β-methoxy-D-homo-17-oxaandrost-4-ene-3,17a-dione was obtained.
¹H-NMR (CDCl₃,δ): 1.27 (6H,s), 3.19 (3H,s), 3.7-3.8 (1H,m), 4.1-4.6 (2H,m), 5.80 (1H,s)
MS (m/z): 332 (M⁺), 317

### Example 33

The same procedure as in Example 3 was conducted except that 200 mg of 6β-methoxy-D-homo-17-oxaandrost-4-ene-3, 17a-dione was used in place of 6β-(acetylthio)-D-homo-17-oxaandrost-4-en-3-one, and the resultant crude product was purified by TLC [developing solvent, chloroform : acetone (8 : 1)] to obtain 57 mg of 6β-methoxy-D-homo-17-oxaandrosta-1,4-diene-3,17a-dione.

### Example 34

The same procedure as in Example 3 was conducted except that 78 mg of 6β-(acetylthio)-D-homo-17-oxaandrost-4-ene-3,17a-dione was used in place of 6β-(acetylthio)-D-homo-17-oxaandrost-4-en-3-one, whereby 1 mg of 6β-(acetylthio)-D-homo-17-oxaandrosta-1,4-diene-3,17a-dione was obtained.
¹H-NMR (CDCl₃,δ): 1.25 (3H,s), 1.31 (3H,s), 2.36 (3H,s), 4.2-4.6 (2H,m), 4.7-4.8 (1H,m), 6.2-6.3 (2H,m), 7.00 (1H,d,J=10Hz)

### Example 35

To a mixture of 100 mg of 3-acetoxy-D-homo-17-oxaandrosta-3,5-dien-17a-one, 1.16 ml of phosphate buffer (pH 8) and 1.16 ml of dioxane was added dropwise under ice cooling over a period of 20 minutes a mixture of 120 mg of m-chloroperbenzoic acid, 1.16 ml of phosphate buffer (pH 8) and 1.16 ml of dioxane. The reaction mixture was stirred at room temperature for 1.5 hours, 50 mg of sodium thiosulfate and 100 mg of sodium hydrogen carbonate were added, and the mixture was stirred for 15 minutes. The reaction mixture was poured in water, and the product was extracted four times with methylene chloride. The extract was washed with saturated saline, and dried over anhydrous magnesium sulfate. The solvent was distilled off to obtain 93 mg of 6ξ-hydroxy-D-homo-17-oxaandrost-4-ene-3,17a-dione.

30 mg thereof was purified by TLC [developing solvent, chloroform : acetone (4 : 1)] to obtain 6 mg of 6α-hydroxy-D-homo-17-oxaandrost-4-ene-3,17a-dione as a high polar compound.
¹H-NMR (CDCl₃,δ): 1.18 (3H,s), 1.26 (3H,s), 4.1-4.7 (3H,m), 6.18 (1H,d,J=1.8Hz)
MS (m/z): 318 (M⁺), 303, 300, 289

Further, 16 mg of 6β-hydroxy-D-homo-17-oxaandrost-4-ene-3,17a-dione was obtained as a low polar compound.
¹H-NMR (CDCl₃,δ): 1.29 (3H,s), 1.38 (3H,s), 4.1-4.7 (3H,m), 5.83 (1H,s)
MS (m/z): 318 (M⁺), 303, 300, 289

### Example 36

A mixture of 63 mg of 6ξ-hydroxy-D-homo-17-oxaandrost-4-ene-3,17a-dione, 0.25 ml of acetic anhydride and 0.5 ml of pyridine was stirred at room temperature for one hour. Water was added to the reaction mixture, and the product was extracted with ethyl acetate. The extract was washed with diluted hydrochloric acid, aqueous saturated sodium hydrogen carbonate solution and saturated saline, and dried over anhydrous magnesium sulfate. The solvent was distilled off, and the resultant crude product was purified by TLC [developing solvent, chloroform : acetone (19 : 1)] to obtain 26 mg of 6β-acetoxy-D-homo-17-oxaandrost-4-ene-3,17a-dione as a high polar compound.
¹H-NMR (CDCl₃,δ): 1.30 (6H,s), 2.05 (3H,s), 4.1-4.7 (2H,m), 5.47 (1H,t,J=3Hz), 5.96 (1H,s)
MS (m/z): 360 (M⁺), 332, 318, 303

Further, 12 mg of 6α-acetoxy-D-homo-17-oxaandrost-4-ene-3,17a-dione was obtained as a low polar compound.
¹H-NMR (CDCl₃,δ): 1.24 (3H,s), 1.26 (3H,s), 2.13 (3H,s), 4.1-4.7 (2H,m), 5.48 (1H,ddd,J=2, 5.5, 12Hz), 5.94 (1H,d,J=2Hz)
MS (m/z): 360 (M⁺), 318, 303

### Example 37

A mixture of 13.5 mg of 6β-acetoxy-D-homo-17-oxaandrost-4-ene-3,17a-dione, 13 mg of DDQ and 0.4 ml of dioxane was refluxed overnight. The insoluble matter was removed from the reaction mixture by filtration, and the resultant solution was purified by alumina column chromatography [eluent, chloroform : acetone (1 : 1)], and further purified by TLC [developing solvent, chloroform : acetone (9 : 1)] to obtain 12 mg of 6β-acetoxy-D-homo-17-oxaandrosta-1,4-diene-3,17a-dione.
¹H-NMR (CDCl₃,δ): 1.34 (6H,s), 2.07 (3H,s), 4.1-4.7 (2H,m), 5.54 (1H,t,J=3Hz), 6.2-6.4 (2H,m), 7.02 (1H,d,J=10Hz)
MS (m/z): 358 (M⁺), 316, 298

### Example 38

The same procedure as in Example 37 was conducted except that 10 mg of 6α-acetoxy-D-homo-17-oxaandrost-4-ene-3,17a-dione was used in place of 6β-acetoxy-D-homo-17-oxaandrost-4-ene-3,17a-dione, whereby 3 mg of 6α-acetoxy-D-homo-17-oxaandrosta-1,4-diene-3,17a-dione was obtained.
¹H-NMR (CDCl₃,δ): 1.29 (6H,s), 2.16 (3H,s), 4.1-4.7 (2H,m), 5.57 (1H,ddd,J=2, 5.5, 12Hz), 6.28 (1H,t,J=2Hz), 6.29 (1H,dd,J=2, 11Hz), 7.01 (1H,d,J=11Hz)
MS (m/z): 358 (M⁺), 316, 298

### Example 39

A mixture of 150 mg of 6α-hydroxy-D-homo-17-oxaandrost-4-ene-3,17a-dione, 15 ml of methyl iodide and 750 mg of silver oxide was refluxed for 10 hours. Chloroform was added to the reaction mixture, and the insoluble matter was removed by filtration. The filtrate was subjected to distillation to remove the solvent, and the resultant crude product was purified by TLC [developing solvent, chloroform : acetone (19 : 1)] to obtain 82 mg of 6α-methoxy-D-homo-17-oxaandrost-4-ene-3,17a-dione.
¹H-NMR (CDCl₃,δ): 1.18 (3H,s), 1.26 (3H,s), 3.41 (3H,s), 3.79 (1H,ddd,J=2, 5, 12Hz), 4.1-4.7 (2H,m), 6.11 (1H,d,J=2Hz)
MS (m/z): 332 (M⁺), 317, 300, 276

### Example 40

The same procedure as in Example 37 was conducted except that 79 mg of 6α-methoxy-D-homo-17-oxaandrost-4-ene-3,17a-dione was used in place of 6β-acetoxy-D-homo-17-oxaandrost-4-ene-3,17a-dione, whereby 43 mg of 6α-methoxy-D-homo-17-oxaandrosta-1,4-diene-3,17a-dione was obtained.
¹H-NMR (CDCl₃,δ): 1.23 (3H,s), 1.29 (3H,s), 3.44 (3H,s), 3.92 (1H,ddd,J=1.5, 5, 11.5Hz) , 4.1-4.7 (2H,m), 6.29 (1H,dd,J=2, 10Hz), 6.39 (1H,t,J=2Hz), 6.99 (1H,d,J=10Hz)
MS (m/z): 330 (M⁺), 315, 271

### Example 41

The same procedure as in Example 39 was conducted except that ethyl iodide was used in place of methyl iodide, whereby 82 mg of 6α-ethoxy-D-homo-17-oxaandrost-4-ene-3,17a-dione was obtained.
¹H-NMR (CDCl₃,δ): 1.18 (3H,s), 1.23 (3H,t,J= 7Hz), 1.26 (3H,s), 3.55 (2H,q,J=7Hz), 3.88 (1H,ddd,J= 2, 5.5, 12Hz), 4.1-4.7 (2H,m), 6.14 (1H,d,J=2Hz)
MS (m/z): 346 (M⁺), 331, 318, 303, 290

### Example 42

The same procedure as in Example 37 was conducted except that 75 mg of 6α-ethoxy-D-homo-17-oxaandrost-4-ene-3,17a-dione was used in place of 6β-acetoxy-D-homo-17-oxaandrost-4-ene-3,17a-dione, whereby 24 mg of 6α-ethoxy-D-homo-17-oxaandrosta- 1,4-diene-3,17a-dione was obtained.
¹H-NMR (CDCl₃,δ): 1.22 (3H,s), 1.26 (3H,t,J= 7Hz), 1.29 (3H,s), 3.57 (2H,br q,J=7Hz), 4.02 (1H,ddd,J= 2, 5, 11.5Hz), 4.1-4.7 (2H,m), 6.29 (1H,dd,J=2, 10Hz), 6.41 (1H,t,J=2Hz), 6.99 (1H,d,J=10Hz)
MS (m/z): 344 (M⁺), 329, 315, 298, 271

### Example 43

The same procedure as in Example 36 was conducted except that 100 mg of 6α-hydroxy-D-homo-17-oxaandrost-4-en-3-one was used in place of 6ξ-hydroxy-D-homo-17-oxaandrost-4-ene-3,17a-dione, whereby 100 mg of 6α-acetoxy-D-homo-17-oxaandrost-4-en-3-one was obtained.
¹H-NMR (CDCl₃,δ): 1.03 (3H,s), 1.24 (3H,s), 2.12 (3H,s), 2.98, 3.42 (2H,ABq,J=11Hz), 3.2-3.5 (1H,m), 3.9-4.2 (1H,m), 5.3-5.6 (1H,m), 5.92 (1H,d,J=2Hz)
MS (m/z): 346 (M⁺), 330, 304, 288

### Example 44

A mixture of 20 mg of D-homo-17-oxaandrosta-1,4-diene-3,6,17a-trione, 3 mg of sodium borohydride and 6 ml of methanol was stirred under ice cooling for 5 minutes. Water was added to the reaction mixture, and the product was extracted with ethyl acetate. The extract was washed with saturated saline, and dried over anhydrous magnesium sulfate. The solvent was distilled off, and the resultant crude product was purified by TLC [developing solvent, chloroform : acetone (9 : 1)] to obtain 4 mg of 6α-hydroxy-D-homo-17-oxaandrosta-1,4-diene-3,17a-dione as a high polar compound.
¹H-NMR (CDCl₃,δ): 1.22 (3H,s), 1.29 (3H,s), 4.1-4.7 (3H,m), 6.29 (1H,dd,J=2, 10Hz), 6.48 (1H,t,J= 2Hz), 7.00 (1H,d,J=10Hz)
MS (m/z): 316 (M⁺), 301, 271

Further, 1 mg of 6β-hydroxy-D-homo-17-oxaandrosta-1,4-diene-3,17a-dione was obtained as a low polar compound.
¹H-NMR (CDCl₃,δ): 1.32 (3H,s), 1.44 (3H,s), 4.1-4.7 (3H,m), 6.1-6.4 (2H,m), 7.03 (1H,d,J=9.5Hz)
MS (m/z): 316 (M⁺), 301, 271

### Example 45

A mixture of 10 mg of D-homo-17-oxaandrosta-1,4-diene-3,6,17a-trione, 100 mg of p-toluenesulfonic acid and 3 ml of methanol was stirred at 40°C for 3 hours. Aqueous saturated sodium hydrogen carbonate solution was added to the reaction mixture, and the product was extracted with ethyl acetate. The extract was washed with saturated saline, and dried over anhydrous magnesium sulfate. The solvent was distilled off, and the resultant crude product was purified by TLC [developing solvent , chloroform : ace tone (19 : 1 )] to obtain 1 mg of 6-methoxy-D-homo-17-oxaandrosta-1,4,6-triene-3,17a-dione.
¹H-NMR (CDCl₃,δ): 1.20 (3H,s), 1.34 (3H,s), 3.65 (3H,s), 4.1-4.7 (2H,m), 5.02 (1H,br d,J=2Hz), 6.30 (1H,dd,J=2, 10Hz), 6.55 (1H,br d,J=2Hz), 7.03 (1H,d,J= 10Hz)

### Example 46

The same procedure as in Example 45 was conducted except that 40 mg of D-homo-17-oxaandrost-4-ene-3,6,17a-trione was used in place of D-homo-17-oxaandrosta-1,4-diene-3,6,17a-trione and ethanol was used in place of methanol, whereby 11 mg of 6-ethoxy-D-homo-17-oxaandrosta-4,6-diene-3,17a-dione was obtained.
¹H-NMR (CDCl₃,δ): 1.12 (3H,s), 1.31 (3H,s), 1.34 (3H,t,J=7Hz), 3.77 (2H,br q,J=7Hz), 4.1-4.7 (2H,m), 5.15 (1H,br), 6.33 (1H,s)

### Example 47

The same procedure as in Example 37 was conducted except that 11 mg of 6-ethoxy-D-homo-17-oxaandrosta-4,6-diene-3,17a-dione was used in place of 6β-acetoxy-D-homo-17-oxaandrost-4-ene-3,17a-dione, whereby 3 mg of 6-ethoxy-D-homo-17-oxaandrosta-1,4,6-triene-3,17a-dione was obtained.
¹H-NMR (CDCl₃,δ): 1.21 (3H,s), 1.34 (3H,s), 1.36 (3H,t,J=7Hz), 3.6-4.0 (2H,m), 4.1-4.7 (2H,m), 5.02 (1H,br d,J=2Hz), 6.30 (1H,dd,J=2, 10Hz), 6.60 (1H,br d,J=2Hz), 7.04 (1H,d,J=10Hz)
MS (m/z): 342 (M⁺), 325, 314

### Example 48

The same procedure as in Example 37 was conducted except that 100 mg of 6α-acetoxy-D-homo-17-oxaandrost-4-en-3-one was used in place of 6β-acetoxy-D-homo-17-oxaandrost-4-ene-3,17a-dione, whereby 67 mg of 6α-acetoxy-D-homo-17-oxaandrosta-1,4-dien-3-one was obtained.
¹H-NMR (CDCl₃,δ): 1.05 (3H,s), 1.29 (3H,s), 2.15 (3H,s), 2.97, 3.41 (2H,ABq,J=10.5Hz), 3.2-3.5 (1H,m), 3.9-4.2 (1H,m), 5.4-5.7 (1H,m), 6.25 (1H,t,J= 2Hz), 6.28 (1H,dd,J=2, 10.5Hz), 7.01 (1H,d,J=10.5Hz)

### Example 49

The same procedure as in Example 44 was conducted except that 40 mg of D-homo-17-oxaandrosta-1,4-diene-3,6-dione was used in place of D-homo-17-oxaandrosta-1,4-diene-3,6,17a-trione, whereby 26 mg of 6α-hydroxy-D-homo-17-oxaandrosta-1,4-dien-3-one was obtained as a high polar compound.
¹H-NMR (CDCl₃,δ): 1.05 (3H,s), 1.22 (3H,s), 2.97, 3.41 (2H,ABq,J=11Hz), 3.2-3.5 (1H,m), 3.9-4.2 (1H,m), 4.3-4.8 (1H,m), 6.27 (1H,dd,J=2, 10Hz), 6.47 (1H,t,J=2Hz), 7.00 (1H,d,J=10Hz)
MS (m/z): 302 (M⁺), 284, 257, 230

Further, 4 mg of 6β-hydroxy-D-homo-17-oxaandrosta-1,4-dien-3-one was obtained as a low polar compound.
¹H-NMR (CDCl₃,δ): 1.09 (3H,s), 1.44 (3H,s), 2.98, 3.41 (2H,ABq,J=11Hz), 3.2-3.5 (1H,m), 3.9-4.2 (1H,m), 4.55 (1H,br), 6.1-6.3 (2H,m), 7.04 (1H,d,J=10Hz) MS (m/z): 302 (M⁺), 257, 230

### Example 50

The same procedure as in Example 45 was conducted except that 10 mg of D-homo-17-oxaandrosta-1,4-diene-3,6-dione was used in place of D-homo-17-oxaandrosta-1,4-diene-3,6,17a-trione and ethanol was used in place of methanol , whereby 1 mg of 6-ethoxy-D-homo-17-oxaandrosta-1,4,6-trien-3-one was obtained.
¹H-NMR (CDCl₃,δ): 1.10 (3H,s), 1.21 (3H,s), 1.35 (3H,t,J=7Hz), 3.00, 3.42 (2H,ABq,J=11Hz), 3.2-4.3 (4H,m), 5.07 (1H,br d,J=2Hz), 6.28 (1H,dd,J=2, 10Hz), 6.58 (1H,br s), 7.03 (1H,d,J=10Hz)

### Example 51

The same procedure as in Example 45 was conducted except that 15 mg of D-homo-17-oxaandrosta-1,4-diene-3,6-dione was used in place of D-homo-17-oxaandrosta-1,4-diene-3,6,17a-trione, whereby 6 mg of 6-methoxy-D-homo-17-oxaandrosta-1,4,6-trien-3-one was obtained.
¹H-NMR (CDCl₃,δ): 1.10 (3H,s), 1.20 (3H,s), 3.00, 3.42 (2H,ABq,J=11Hz), 3.2-3.5 (1H,m), 3.63 (3H,s), 4.0-4.3 (1H,m), 5.08 (1H,br d,J=2Hz), 6.28 (1H,dd,J= 2,10Hz), 6.52 (1H,br s), 7.03 (1H,d,J=10Hz)

### Example 52

The same procedure as in Example 45 was conducted except that 50 mg of D-homo-17-oxaandrost-4-ene-3,6-dione was used in place of D-homo-17-oxaandrosta-1,4-diene-3,6, 17a-trione, whereby 40 mg of 6-methoxy-D-homo-17-oxaandrosta-4,6-dien-3-one was obtained.
¹H-NMR (CDCl₃,δ): 1.08 (3H,s), 1.12 (3H,s), 3.02, 3.43 (2H,ABq,J=11Hz), 3.2-3.5 (1H,m), 3.59 (3H,s), 4.0-4.3 (1H,m), 5.21 (1H,br d,J=2.5Hz), 6.25 (1H,s)

### Example 53

The same procedure as in Example 37 was conducted except that 26 mg of 6-methoxy-D-homo-17-oxaandrosta-4,6-dien-3-one was used in place of 6β-acetoxy-D-homo-17-oxaandrost-4-ene-3,17a-dione, whereby 16 mg of 6-methoxy-D-homo-17-oxaandrosta-1,4,6-trien-3-one was obtained.

### Example 54

The same procedure as in Example 44 was conducted except that 4.5 g of D-homo-17-oxaandrost-4-ene-3,6-dione was used in place of D-homo-17-oxaandrosta-1,4-diene-3,6,17a-trione, and the resultant crude product was recrystallized from acetone-ether to obtain 2.4 g of 6α-hydroxy-D-homo-17-oxaandrost-4-en-3-one.
¹H-NMR (CDCl₃,δ): 1.02 (3H,s), 1.18 (3H,s), 2.99, 3.41 (2H,ABq, J=11Hz), 3.2-3.5 (1H,m), 3.9-4.5 (2H,m), 6.17 (1H,d,J=2Hz)
MS (m/z): 304 (M⁺), 289, 275, 235

### Example 55

The same procedure as in Example 39 was conducted except that 50 mg of 6α-hydroxy-D-homo-17-oxaandrost-4-en-3-one was used in place of 6α-hydroxyD-homo-17-oxaandrost-4-ene-3,17a-dione, whereby 31 mg of 6α-methoxy-D-homo-17-oxaandrost-4-en-3-one was obtained.
¹H-NMR (CDCl₃,δ): 1.02 (3H,s), 1.18 (3H,s), 2.99 (1H,d,J=11Hz), 3.2-3.6 (2H,m), 3.40 (3H,s), 3.77 (1H,ddd,J=2, 5.5, 12Hz), 3.9-4.2 (1H,m), 6.10 (1H,d,J=2Hz)
MS (m/z): 318 (M⁺), 303, 262

### Example 56

The same procedure as in Example 37 was conducted except that 29 mg of 6α-methoxy-D-homo-17-oxaandrost-4-en-3-one was used in place of 6β-acetoxy-D-homo-17-oxaandrost-4-ene-3,17a-dione, whereby 19 mg of 6α-methoxy-D-homo-17-oxaandrosta-1,4-dien-3-one was obtained.
¹H-NMR (CDCl₃,δ): 1.05 (3H,s), 1.23 (3H,s), 2.96, 3.40 (2H,ABq,J=11Hz), 3.2-3.5 (1H,m), 3.43 (3H,s), 3.7-4.2 (2H,m), 6.27 (1H,dd,J=2, 10Hz), 6.38 (1H,t,J= 2Hz), 6.99 (1H,d,J=10Hz)
MS (m/z): 316 (M⁺), 300, 284, 257

### Example 57

The same procedure as in Example 39 was conducted except that 50 mg of 6α-hydroxy-D-homo-17-oxaandrost-4-en-3-one was used in place of 6α-hydroxy-D-homo-17-oxaandrost-4-ene-3,17a-dione and ethyl iodide was used in place of methyl iodide, whereby 40 mg of 6α-ethoxy-D-homo-17-oxaandrost-4-en-3-one was obtained.
¹H-NMR (CDCl₃,δ): 1.02 (3H,s), 1.17 (3H,s), 1.22 (3H,t,J=7Hz), 2.98 (1H,d,J=11Hz), 3.2-4.2 (6H,m), 6.13 (1H,d,J=2Hz)
MS (m/z): 332 (M⁺), 317, 276

### Example 58

The same procedure as in Example 37 was conducted except that 36 mg of 6α-ethoxy-D-homo-17-oxaandrost-4-en-3-one was used in place of 6β-acetoxy-D-homo-17-oxaandrost-4-ene-3,17a-dione, whereby 24 mg of 6α-ethoxy-D-homo-17-oxaandrosta-1,4-dien-3-one was obtained.
¹H-NMR (CDCl₃,δ): 1.05 (3H,s), 1.22 (3H,s), 1.25 (3H,t,J=7Hz), 2.97, 3.40 (2H,ABq,J=11Hz), 3.2-3.6 (1H,m), 3.56 (2H,q, J=7Hz), 3.9-4.2 (2H,m), 6.27 (1H,dd,J=2, 10Hz), 6.40 (1H,t,J=2Hz), 7.00 (1H,d,J=10Hz)
MS (m/z): 330 (M⁺), 284, 257

### Preparation example 1

A mixture of 20 g of D-homo-17-oxaandrost-4-en-3-one, 40 ml of ethylene glycol , 80 ml of ethyl orthoformate and 2.4 g of p-toluenesulfonic acid was stirred at room temperature for one hour. The reaction mixture was poured in 1.5 ℓ of water containing 20 ml of pyridine, and the crystals deposited were taken by filtration. The crystals were air-dried to obtain 22.1 g of 3,3-(ethylenedioxy)-D-homo-17-oxaandrost-5-ene.
¹H-NMR (CDCl₃,δ): 1.00 (3H,s), 1.03 (3H,s), 2.98, 3.38 (2H,ABq,J=11Hz), 3.2-3.5 (1H,m), 3.8-4.2 (5H,m), 5.3-5.4 (1H,m)

### Preparation example 2

A mixture of 20 g of 3,3-(ethylenedioxy)-D-homo-17-oxaandrost-5-ene, 200 ml of chloroform and 20.8 g of m-chloroperbenzoic acid was stirred under ice cooling for 20 minutes. Aqueous saturated sodium hydrogen carbonate solution was added to the reaction mixture, and the product was extracted with ethyl acetate. The extract was washed with aqueous 5 % sodium hydroxide solution and saturated saline, and dried over anhydrous magnesium sulfate. The solvent was distilled off, and the resultant crude product was purified by silica gel column chromatography [eluent, benzene : ethyl acetate (20 : 1)] to obtain 13.1 g of 5,6α-epoxy-3,3-(ethylenedioxy)-D-homo-17-oxa-5α-androstane.
¹H-NMR (CDCl₃,δ): 0.93 (3H,s), 1.07 (3H,s), 2.84 (1H,d,J=4.2Hz), 2.94, 3.34 (2H,ABq,J=11Hz), 3.2-3.5 (1H,m), 3.8-4.1 (5H,m)
MS (m/z): 348 (M⁺)

### Preparation example 3

A mixture of 2.1 g of D-homo-17-oxaandrost-4-ene-3-one, 1.5 g of N-bromosuccinimide, 20 ml of carbon tetrachloride and 1 mg of azobisisobutyronitrile was refluxed for 2 hours. The insoluble matter was removed from the reaction mixture by filtration, and the solvent was distilled off. The resultant crude product was purified by TLC [developing solvent, chloroform : ethyl acetate (40 : 1)] to obtain 6ξ-bromo-D-homo-17-oxaandrost-4-en-3-one.

### Preparation example 4

A mixture of 35.4 g of 3β-hydroxy-D-homo-17-oxaandrost-5-en-17a-one, 106 ml of tetrahydrofuran, 35 ml of ethylene glycol, 70 ml of ethyl orthoformate and 2.8 g of p-toluenesulfonic acid was stirred at 50°C for 1.5 hours. To this reaction mixture were added 42 ml of aqueous 10 % potassium hydroxide solution and 354 ml of 28 % solution of sodium methylate in methanol, and the mixture was stirred at that temperature for 3 hours. After being left alone to cool , the reaction mixture was poured in 4.2 ℓ of water. The crystals deposited were taken by filtration, washed, and dried to obtain 38.9 g of 17a, 17a-(ethylenedioxy)-D-homo-17-oxaandrost-5-en-3β-ol.
¹H-NMR (CDCl₃,δ): 1.00 (3H,s), 1.10 (3H,s), 3.2-4.3 (7H,m), 5.34 (1H,br d,J=5Hz)

### Preparation example 5

A mixture of 38.9 g of 17a,17a-(ethylenedioxy)-D-homo-17-oxaandrost-5-en-3β-ol, 80 g of magnesium monoperphthalate hexahydrate and 400 ml of dimethylformamide was stirred at 60°C for 40 minutes. The reaction mixture was left alone to cool, water was added thereto, and the mixture was neutralized with aqueous 10 % sodium hydroxide solution. The crystals deposited were taken by filtration, washed with water and dried. The resultant crude product was recrystallized from acetone-hexane to obtain 20.6 g of 5,6α-epoxy-17a,17a-(ethylenedioxy)-D-homo-17-oxa-5α-androstan-3β-ol.
¹H-NMR (CDCl₃,δ): 1.04 (6H,s), 2.91 (1H,d,J=4Hz), 3.5-4.2 (7H,m)
MS (m/z): 365 (MH⁺), 349, 319

### Preparation example 6

The same procedure as in Example 1 (a) and (b) was conducted except that 5,6α-epoxy-17a,17a-(ethylenedioxy)-D-homo-17-oxa-5α-androstan-3β-ol was used in place of 5,6α-epoxy-3,3-(ethylenedioxy)-D-homo-17-oxa-5α-androstane, whereby 592 mg of 6β-(acetylthio)-3β,5-dihydroxy-D-homo-17-oxa-5α-androstan-17a-one was obtained.
¹H-NMR (CDCl₃,δ): 1.05 (3H,s), 1.22 (3H,s), 2.33 (3H,s), 3.3-3.5 (1H,m), 3.6-3.8 (1H,m), 3.9-4.6 (4H,m)

### Preparation example 7

A mixture of 592 mg of 6β-(acetylthio)-3β,5-dihydroxy-D-homo-17-oxa-5α-androstan-17a-one, 5 ml of acetone and 1 ml of Jones' reagent was stirred under ice cooling for 1 minute. Water was added to the reaction mixture, and the product was extracted with ethyl acetate. The extract was washed with aqueous saturated sodium hydrogen carbonate solution and saturated saline, and dried over anhydrous sodium sulfate. The solvent was distilled off to obtain 450 mg of 6β-(acetylthio)-5-hydroxy-D-homo-17-oxa-5α-androstane-3,17a-dione.
¹H-NMR (CDCl₃,δ): 1.25 (6H,s), 2.35 (3H,s), 3.3-3.5 (1H,m), 3.6-3.8 (1H,m), 4.1-4.5 (2H,m)

### Preparation example 8

A mixture of 2 g of D-homo-17-oxaandrosta-1,4-diene-3,17a-dione, 2 g of N-bromosuccinimide, 100 mg of benzoyl peroxide and 20 ml of carbon tetrachloride was refluxed for 5 hours. The reaction mixture was left alone to cool, the insoluble matter was removed therefrom, and the solvent was distilled off under reduced pressure. The resultant crude product was purified by silica gel column chromatography [eluent, hexane : ethyl acetate (1 : 1)] to obtain 1.1 g of 6β-bromo-D-homo-17-oxaandrosta-1,4-diene-3,17a-dione.
¹H-NMR (CDCl₃,δ): 1.35 (3H,s), 1.57 (3H,s), 4.0-4.7 (2H,m), 5.0-5.1 (1H,m), 6.15-6.30 (2H,m), 7.04 (1H,d,J=10.5Hz)
MS (m/z): 380, 378 (M⁺), 365, 363

### Preparation example 9

The same procedure as in Preparation example 8 was conducted except that 3.3 g of D-homo-17-oxaandrosta-1,4-dien-3-one was used in place of D-homo-17-oxaandrosta-1,4-diene-3,17a-dione, and the resultant crude product was purified by silica gel column chromatography [eluent, benzene : ethyl acetate (9 : 1)] to obtain 2.54 g of 6β-bromo-D-homo-17-oxaandrosta-1,4-dien-3-one.
¹H-NMR (CDCl₃,δ): 1.12 (3H,s), 1.58 (3H,s), 2.98, 3.43 (2H,ABq,J=11Hz), 3.2-3.5 (1H,m), 3.9-4.2 (1H,m), 5,05 (1H,dd,J=2.2, 4.2Hz), 6.16-6.28 (2H,m), 7.05 (1H,d,J=11Hz)
MS (m/z): 366, 364 (M⁺), 351 , 349

### Preparation example 10

To a mixture of 100 mg of 5,6α-epoxy-3,3-(ethylenedioxy)-D-homo-17-oxa-5α-androstane, 0.64 ml of tetrahydrofuran and 0.5 ml of methanol was added under ice cooling 0.61 ml of 3 N aqueous perchloric acid, and the mixture was stirred for 15 minutes. Water was added to the reaction mixture, and the product was extracted with ethyl acetate. The extract was washed with aqueous saturated sodium hydrogen carbonate solution and saturated saline, and dried over an hydrous sodium sulfate. The solvent was distilled off to obtain 113 mg of 5-hydroxy-6β-methoxy-D-homo-17-oxa-5α-androstan-3-one.
¹H-NMR (CDCl₃,δ): 1.01 (3H,s), 1.25 (3H,s), 2.9-3.1 (1H,m), 2.98, 3.39 (2H,ABq,J=11Hz), 3.28 (3H,s), 3.2-3.4 (1H,m), 3.9-4.2 (1H,m)
MS (m/z): 336 (M⁺), 318, 303

### Preparation example 11

A mixture of 110 mg of 5-hydroxy-6β-methoxy-D-homo-17-oxa-5α-androstan-3-one, 3.0 ml of acetic acid and 0.2 ml of 1 N solution of bromine in acetic acid was stirred at room temperature for 30 minutes. Water was added to the reaction mixture, and the product was extracted with ethyl acetate. The extract was washed with aqueous saturated sodium hydrogen carbonate solution and saturated saline, and dried over anhydrous sodium sulfate. The solvent was distilled off, and the resultant crude product was purified by TLC [developing solvent, chloroform : acetone (19 : 1)] to obtain 43 mg of 2α-bromo-5-hydroxy-6β-methoxy-D-homo-17-oxa-5α-androstan-3-one.
¹H-NMR (CDCl₃,δ): 1.01 (3H,s), 1.31 (3H,s), 2.99, 3.39 (2H,ABq,J=11Hz), 3.28 (3H,s), 3.2-3.5 (1H,m), 4.0-4.2 (1H,m), 4.76 (1H,t,J=10Hz)
MS (m/z): 416, 414 (M⁺), 398, 396, 383, 381, 367, 365, 335

### Preparation example 12

The same procedure as in Preparation example 5 was conducted except that 5 g of 3β-hydroxy-D-homo-17-oxaandrost-5-en-17a-one was used in place of 17a, 17a-(ethylenedioxy)-D-homo-17-oxaandrost-5-en-3β-ol, whereby 2.56 g of 5,6α-epoxy-3β-hydroxy-D-homo-17-oxa-5α-androstan-17a-one was obtained.

### Preparation example 13

The same procedure as in Preparation example 10 was conducted except that 300 mg of 5,6α-epoxy-3β-hydroxy-D-homo-17-oxa-5α-androstan-17a-one was used in place of 5,6α-epoxy-3,3-(ethylenedioxy)-D-homo-17-oxa-5α-androstane, whereby 214 mg of 3β,5-dihydroxy-6β-methoxy-D-homo-17-oxa-5α-androstan-17a-one was obtained.

### Preparation example 14

The same procedure as in Preparation example 10 was conducted except that 1 g of 5,6α-epoxy-17a,17a-(ethylenedioxy)-D-homo-17-oxa-5α-androstan-3β-ol was used in place of 5,6α-epoxy-3,3-(ethylenedioxy)-D-homo-17-oxa-5α-androstane, whereby 0.7 g of 3β,5-dihydroxy-6β-methoxy-D-homo-17-oxa-5α-androstan-17a-one was obtained.

### Preparation example 15

A mixture of 214 mg of 3β,5-dihydroxy-6β-methoxy-D-homo-17-oxa-5α-androstan-17a-one, 2 ml of acetone and 0.4 ml of Jones' reagent was stirred under ice cooling for 10 minutes. To the reaction mixture was added 5 ml of isopropyl alcohol, and the mixture was stirred for 5 minutes, diluted with ethyl acetate, washed with aqueous saturated sodium hydrogen carbonate solution and saturated saline, and dried over anhydrous sodium sulfate. The solvent was distilled off to obtain 122 mg of 5-hydroxy-6β-methoxy-D-homo-17-oxa-5α-androstane-3,17a-dione.

### Preparation example 16

The same procedure as in Preparation example 11 was conducted except that 122 mg of 5-hydroxy-6β-methoxy-D-homo-17-oxa-5α-androstane-3,17a-dione was used in place of 5-hydroxy-6β-methoxy-D-homo-17-oxa-5α-androstan-3-one, and the resultant crude product was purified by TLC [developing solvent, chloroform : acetone (10 : 1)] to obtain 14 mg of 2α-bromo-5-hydroxy-6β-methoxy-D-homo-17-oxa-5α-androstan-3,17a-dione.
¹H-NMR (CDCl₃,δ): 1.24 (3H,s), 1.30 (3H,s), 3.0-3.1 (1H,m), 3.30 (3H,s), 4.2-4.6 (2H,m), 4.6-4.9 (1H,m)
MS (m/z): 430, 428 (M⁺), 349, 331

### Preparation example 17

A mixture of 10 g of D-homo-17-oxaandrost-4-ene-3,17a-dione, 18.5 ml of acetic anhydride, 0.3 ml of pyridine and 29.5 ml of acetyl chloride was refluxed for 2.5 hours. The solvent was distilled off from the reaction mixture under reduced pressure, 50 ml of cold ethanol was added to the resultant crude product under ice cooling, and the mixture was stirred for 15 minutes. The crystals deposited were taken by filtration and dried to obtain 7.1 g of 3-acetoxy-D-homo-17-oxaandrosta-3,5-dien-17a-one.
¹H-NMR (CDCl₃,δ): 1.00 (3H,s), 1.25 (3H,s), 2.13 (3H,s), 4.0-4.7 (2H,m), 5.3-5.5 (1H,m) , 5.71 (1H,m)
MS (m/z): 344 (M⁺), 316, 302

### Preparation example 18

The same procedure as in Example 37 was conducted except that 900 mg of D-homo-17-oxaandrost-4-ene-3,6,17a-trione was used in place of 6β-acetoxy-D-homo-17-oxaandrost-4-ene-3,17a-dione, whereby 41 mg of D-homo-17-oxaandrosta-1,4-diene-3,6,17a-trione was obtained.
¹H-NMR (CDCl₃,δ): 1.21 (3H,s), 1.31 (3H,s), 4.1-4.7 (2H,m), 6.2-6.5 (2H,m), 7.06 (1H,d,J=10Hz)
MS (m/z): 314 (M⁺), 286

### Preparation example 19

The same procedure as in Example 37 was conducted except that 670 mg of D-homo-17-oxaandrost-4-ene-3,6-dione was used in place of 6β-acetoxy-D-homo-17-oxaandrost-4-ene-3,17a-dione, whereby 100 mg of D-homo-17-oxaandrosta-1,4-diene-3,6-dione was obtained.
¹H-NMR (CDCl₃,δ): 1.08 (3H,s), 1.21 (3H,s), 3.01 , 3.45 (2H,ABq, J=11Hz), 3.2-3.5 (1H,m), 3.9-4.2 (1H,m), 6.2-6.5 (2H,m), 7.07 (1H,d,J=10Hz)
MS (m/z): 300 (M⁺), 272

## Claims

1. A steroid compound represented by the formula 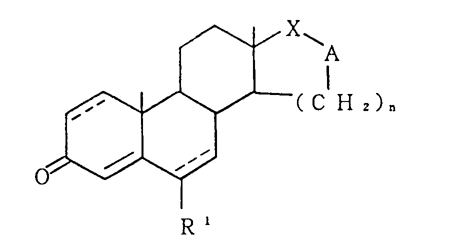 wherein
R¹ represents -S-R², -O-R³ , an azido group or an amino group, and herein
R² represents a hydrogen atom, a lower alkyl group, a cyano group or an acyl group, and
R³ represents a hydrogen atom, a lower alkyl group or an acyl group;
X represents C = O or CH₂,
A represents O or NH,
n represents 1 or 2, and
the broken line between the 1- and
2-positions of the steroid skeleton means that a double bond can optionally exist there, the broken line between the 6- and 7-positions means that a double bond can optionally exist when R¹ is -O-R³ and R³ represents a lower alkyl group, and in other cases than the above, the bond between the 6- and 7-positions is meant to be a single bond,
provided that when A represents NH, X is assumed to represent C = O.

2. The steroid compound according to claim 1 wherein A represents O and n represents 2.

3. The steroid compound according to claim 1 wherein the bond between the 6- and 7-positions of the steroid skeleton is a single bond.

4. The steroid compound according to claim 1 wherein a double bond exists between the 1- and 2-positions of the steroid skeleton.

5. The steroid compound according to claim 1 wherein R¹ represents a lower alkylthio group, an acylthio group, a lower alkoxy group, an acyloxy group or an azido group.

6. An aromatase inhibitor containing a compound according to claim 1.

7. A pharmaceutical composition comprising a compound according to claim 1 and pharmaceutically acceptable additive(s).

8. Use of the composition according to claim 7 for prophylaxis or treatment of a disease caused by estrogens.

9. A method for preventing or treating a disease caused by estrogens in a human being or another mammal, which comprises administering a compound according to claim 1 to the human being or the mammal.
